# EUROPEAN PATENT APPLICATION

(11) **EP 4 382 115 A1**
(43) Date of publication of application: **12.06.2024**
(21) Application number: 22853439.2
(22) Date of filing: 02.08.2022
(51) Int. Cl.: A61K 38/16, A61K 45/06, A61P 43/00

(54) **COMPOSITION FOR PREVENTING OR TREATING FIBROTIC DISEASES, COMPRISING HAPLN1**

(30) Priority: 03.08.2021 KR 20210101726
(71) Applicant: Chung Ang University Industry Academic Cooperation Foundation, Seoul 06974 (KR); HAPLNSCIENCE INC., Gyeonggi-do 13494 (KR)
(72) Inventor: KIM, Dae Kyong, Seongnam-si, Gyeonggi-do 13547 (KR); KIM, Yong Soon, Seoul 04778 (KR); BACK, Moon Jung, Seongnam-si, Gyeonggi-do 13489 (KR); PYO, Jung Hoon, Seongnam-si, Gyeonggi-do 13504 (KR); KIM, David, Yongin-si, Gyeonggi-do 16842 (KR); PIAO, Yong Wei, Suwon-si, Gyeonggi-do 16447 (KR); YEOM, Min A, Suwon-si, Gyeonggi-do 16421 (KR)
(74) Representative: Mewburn Ellis LLP
(86) International application number: PCT/KR2022/011419
(87) International publication number: WO 2023/014062

(57) **Abstract**

Disclosed in the present disclosure are, a pharmaceutical composition for preventing or treating a fibrotic disease including a hyaluronan and proteoglycan link protein 1 (HAPLN1) or a gene encoding the same as an active ingredient, and a method for preventing or treating the fibrotic disease using the composition. According to the present disclosure, by preventing and inhibiting fibrosis of cells or tissues, the development or progression of various diseases caused by fibrosis may be fundamentally inhibited, thereby preventing or treating such diseases.

## Description

### Technical Field

The present disclosure relates to a composition for repairing various tissue damage caused by fibrosis and preventing or treating fibrotic diseases, including hyaluronan and proteoglycan link protein 1 (HAPLN1) or the gene encoding the same as an active ingredient. Specifically, the present disclosure relates to a composition for the repair and regeneration of tissue fibrosis, damage caused by fibrosis, including HAPLN1 as an active ingredient. In addition, the present disclosure relates to a composition for preventing or treating various tissue damage and loss of function caused by fibrosis, and for the prevention or treatment of aging degenerative disease, including a fibrotic disease.

Furthermore, the present disclosure discloses a composition for preventing or treating fibrotic diseases and a method of preventing or inhibiting fibrosis of cells using the composition. According to the present disclosure, by preventing and inhibiting cellular fibrosis, it presents the possibility of fundamentally inhibiting the occurrence or progression of various diseases caused by intervening cellular fibrosis as well as treating them.

### Background Art

The medical definition of 'pulmonary fibrosis', in which the lung hardens like a stone, refers to a disease in which healthy lung tissue changes into scar tissue due to some cause, causing the lung tissue to become thick or hard. As in the humidifier disinfectant incident that occurred in Korea, the cause is clearly known to be a specific harmful chemical substance used to maintain the sterilizing effect in the humidifier, but 'idiopathic pulmonary fibrosis (IPF)' is a disease in which the lungs harden without a clear cause. This disease is a type of interstitial lung disease (ILD) that shows usual interstitial pneumonia (UIP) symptoms, histologically characterized by inflammation and fibrosis of the lung parenchyma, and various risk factors such as aging, smoking, gastroesophageal reflux disease, environmental occupational exposure, dust work, radiation exposure, autoimmunity, drug addiction, hypersensitivity pneumonitis, viral·pathogenic infection, family history, and the like are known. However, despite recent advanced medical technology and the effort of scientists, the pathogenesis of this disease is not clearly known. The average age of onset is 69 years, and the disease mainly occurs in the elderly, with twice or more often in men than in woman, and the prognosis is poor. In the case of 'idiopathic pulmonary fibrosis', a typical type of pulmonary fibrosis, the number of patients in Korea is 1.7 per 100,000 people, and as the cause is unknown, idiopathic pulmonary fibrosis is a very scary disease that is difficult to treat. The average survival period is 60 months, but acute exacerbation due to infection, etc., occurs in 14 % of patients per year. When an acute exacerbation occurs, the survival period drops dramatically to 15 months.

In patients with pulmonary fibrosis, the alveolar walls are thickened with scar tissue, and as this scar tissue gradually worsens, the lung parenchyma tissue is destroyed. At this time, breathing in air becomes especially difficult, making it impossible to breathe in enough air to supply oxygen to the blood. Eventually, alveoli become unable to perform its original function, and shortness of breath, frequent dry coughing, fatigue, and characteristic club-shaped changes appear in the fingertips and toes, and the three main symptoms are coughing, exertional dyspnea, and crackling due to phlegm. The COVID-19 virus, which has recently become a pandemic, may also cause pulmonary fibrosis during the healing process when infection and inflammation occur in the lungs.

The most well-known risk factor of fibrosis is aging. In particular, researchers exploring lung tissue lesions have recently discovered that lung cells in patients with pulmonary fibrosis are unable to sustain division and growth, which are signs of cellular senescence. According to the cellular senescence theory, the aging of organisms, including humans, is believed to be due to the accumulation of senescent cells that are not physiologically useful but rather promote pathological conditions, and aging is a phenomenon in which cells enter a series of processes called cell cycle arrest and no longer divide and produce cells at a normal rate. According to a recent report, the effect of cellular senescence on fibrosis in elderly pneumonia has been systematically described (Yanagi 2017, Int. J. Mol. Sci. 18, 503).

Intracellular DNA damage and chromosome 'end segment wear', in other words telomere attrition, that occur due to smoking or aging occur along with the immunosenescence phenomenon associated with aging, which not only limits the removal of senescent cells present in the body but also as cells no longer proliferate and fail to increase in number (cell growth arrest), they secrete inflammation aging-inducing substance called senescence-associated secretory phenotype (SASP), such as interleukin 1 beta (IL-1β), outside the cells, which affects surrounding normal cells and turns them into senescent cells. As the number of senescent cells gradually increases, more SASP is secreted, leading to a pathological state accompanied by chronic low-grade inflammation, tissue regeneration and repair ability naturally gradually decreases, and eventually the normal lung tissue structure begins to be destroyed, especially in the case of the elderly, as their various comorbidities are added, their resistance to various external pathogenic attacks is reduced (high vulnerability), and the fibrosis that was initially occurring further develops and worsens.

That is, the inventors of the present disclosure note that, as mentioned above, the risk factor for pulmonary fibrosis is old age, and while exploring through qualitative and quantitative analysis techniques using heterochronic parabiosis and proteomics technology between young and old mice to discover 'endogenous substances in the blood' that can prevent or treat the onset of this disease, hyaluronan and proteoglycan link protein 1 (HAPLN1) came into focus as the most promising substance. According to various literature, HAPLN1 protein is one of the constituent proteins of the extracellular matrix (ECM), first discovered in vertebrate cartilage, and is reported to act as a hinge that stabilizes the structural aggregates of hyaluronic acid and proteoglycans, as well as ensuring that the gaps between the proteoglycans are very tightly spaced, creating a bottle-brush shape. The inventors of the present disclosure have demonstrated that the recombinant human protein HAPLN1 (rhHAPLN1) may actually reduce the production of low molecular weight HA (LMW HA) or 32-mer peptide as a damage-associated molecular pattern (DAMP) substance by inhibiting the decomposition of its partner molecules, high molecular weight HA (HMW HA) and aggrecan (a type of proteoglycan), through its hinge-type link function.

Myofibroblasts are the primary cells that excessively form fibrous tissues such as collagen in a fibrotic disease. These myofibroblasts characteristically express the expression of α smooth muscle actin (αSMA), and therefore the level of αSMA expression is an important indicator of the level of expression of myofibroblasts. In fact, the number of myofibroblasts expressing αSMA increases in various fibrotic diseases such as idiopathic pulmonary fibrosis and kidney fibrosis, and an increase in αSMA expression has also been reported in in vivo fibrosis models. These myofibroblasts are mainly derived from fibroblasts, but are also reported to be derived from endothelial cells, epithelial cells, and stem cells (The American Journal of Pathology,. 2007. 170(6): 1807-1816). In the case of liver fibrosis, hepatic stellate cells are activated to form excessive amounts of collagen and other fibrous tissue, and αSMA expression is increased upon activation of hepatic stellate cells, making the expression an indicator of stellate cell activation (Cells 2019, 8(11), 1419).

The most well-known factor that induces differentiation of myofibroblasts (or activation of hepatic stellate cells) is TGFβ1, and in addition, mechanical stress in a stiff environment has been reported to be a factor in the differentiation of myofibroblasts (The American Journal of Pathology,. 2007. 170(6): 1807-1816). Therefore, inducing αSMA expression by TGFβ1 in various cells is used as an in vitro fibrosis model, and its activity in reducing induced αSMA expression is judged to have anti-fibrotic efficacy (Molecular Medicine 2021 27:22).

To date, various animal models have been established to prove the effectiveness of pulmonary fibrosis, but the most widely recognized and used model is the 'inhaled bleomycin (bleomycin, BL) induced pulmonary fibrosis (bleomycin-induced pulmonary fibrosis, BIPF) mouse model'. Originally, bleomycin was used as an anticancer drug or as a treatment for warts, but the three-dose bleomycin (BL)-hamster model established by Professor Giri's group at the University of California, Davis, School of Veterinary Medicine in 1998 (Iyer 1998), the once-daily intravenous mouse model established by Oku et al. in 2008 (Oku 2008, European Journal of Pharmacology 590;400-408), and more recently, Song et al. (EXPERIMENTAL AND THERAPEUTIC MEDICINE 16: 1800-1806, 2018) established a model in which the licensed drug pirfenidone (PFD) was administered orally once daily for 14 or 28 days starting the day after a single bronchial route administration of bleomycin in a rat model to successfully induce pulmonary fibrosis and to study the efficacy of the drug as well as some of its mechanisms. As a result, it was reported that the levels of periostin and TGFβ1, which had been increased by bleomycin, decreased in the 14-day and 28-day groups (Song 2018).

In fact, there are currently only two IPF treatments that have been approved for sale using this IPF mouse model, one of them, pirfenidone (PFD; brand name Pirespa), was first approved by Shionogi & Co., Ltd. in Japan in 2008, followed by the European Union in 2011, Canada in 2012, and the United States in 2014, respectively. Another, nintedanib, trade name Ofev or Vargatef, is a treatment for pulmonary fibrosis that was recently approved in the United States in March 2020. According to Oku et al. (Oku 2008), who developed pirfenidone (PFD), found that when bleomycin was administered IV to mice continuously five times daily, inflammation reached its peak around 10 days after the start of administration, and then the inflammatory response gradually decreased, and fibrosis gradually progressed from about 8 days to 9 days after the start of administration.

More specifically, in order to examine the efficacy of candidate drug pirfenidone on pulmonary fibrosis, the research team administered pirfenidone three times daily for 10 days or 28 days starting the day (day 1) after the first administration of bleomycin (day 0) and then examined its therapeutic efficacy. According to Oku et al., in the group treated simultaneously with prednisolone, a type of anti-inflammatory corticosteroid agent, the levels of inflammation-related biomarkers decreased, but there was no decrease in the level of TGFβ1, a measure of anti-fibrotic efficacy. Therefore, inferring from this, it is concluded that not only is the role of inflammation in the fibrosis process not critical, but also that a rapid inflammatory response may begin immediately after bleomycin administration, and that initial drug administration may also slow down or weaken the fibrosis process that progresses slowly thereafter.

Meanwhile, du Bois et al. came to similar conclusions in a 2010 paper published two years after Oku et al. (du Bois, R. M. Strategies for treating idiopathic pulmonary fibrosis. Nature Rev. Drug Discov. 2010, 9, 129-140).

Through these literature evidence, the inventors of the present disclosure discovered that HAPLN1 protein compositions exhibit excellent efficacy in preventing and treating fibrosis of various tissues, even at very low concentrations using the TGFβ1-induced fibrotic disease cell model and the bleomycin-induced pulmonary fibrosis mouse model (BIPF), etc., as described above, and thus completed the present disclosure.

Regarding HAPLN1, for example, the U.S. Patent Publication 2013/0052198 discloses HAPLN1 polypeptide as one of a wide range of individual factors secreted by marrow stromal cells (MSC) and suggests that when administered to a subject with an inflammatory disease, HAPLN1 may attenuate the characteristics of the disease. However, there is no mention at all about the action of preventing or treating fibrosis in various tissues.

If it is possible to directly prevent or inhibit the abnormal excessive occurrence of fibrosis in cells in vivo, fibrosis would be a valuable countermeasure against a variety of diseases in which fibrosis itself contributes to the pathogenesis or promotes the progression of an already established disease, and there is always a demand for this.

### Disclosure

### Technical Problem

The present disclosure provides compositions for the prevention and treatment of fibrotic diseases including hyaluronan and proteoglycan link protein 1 (HAPLN1) or the gene encoding HAPLN1 as an active ingredient, and methods for the prevention and treatment of fibrotic diseases using the same.

In addition, by providing a reagent composition including hyaluronan and proteoglycan link protein 1 (HAPLN1) or the gene encoding HAPLN1 as an active ingredient, the reagent composition is intended to be used in research on the disease and contribute to identifying the mechanism of the disease and developing compositions for preventing or treating related diseases.

### Technical Solution

In order to solve the above problems, the present disclosure provides the following aspects of the disclosure.
[1] One aspect of the present disclosure relates to a pharmaceutical composition for the prevention or treatment of a fibrotic disease, including as an active ingredient hyaluronan and proteoglycan link protein 1 (HAPLN1) or a gene encoding HAPLN1.
[2] Another aspect of the present disclosure relates to a composition wherein the protein of the composition has at least 80 % sequence identity to the amino acid sequence of SEQ ID NO: 1.
[3] Another aspect of the present disclosure relates to a composition wherein a nucleic acid of the gene of the composition is included in an expression vector.
[4] Another aspect of the present disclosure relates to a composition wherein a foci of fibrotic disease is selected from the group consisting of skin, liver, intestine, heart, lung, and kidney.
[5] Another aspect, of the present disclosure relates to a composition wherein the foci of the fibrotic disease is selected from the group consisting of a skin fibroblast, hepatic stellate cell, colon fibroblast, cardiac microvascular endothelial cell, lung fibroblast, kidney renal tubule cell, and renal proximal tubule epithelial cell.
[6] Another aspect of the present disclosure relates to a composition wherein the fibrotic disease is ischemic fibrosis.
[7] Another aspect of the present disclosure relates to a composition wherein a single dose of the composition is from 0.1 ng/ml to 500 ng/ml.
[8] Another aspect of the present disclosure relates to a composition wherein the composition is administered at a dose of 0.001 mg/kg BWto 5 mg/kg BW of rhHAPLN1 protein when administered in vivo.
[9] Another aspect of the present disclosure relates to a composition wherein the composition is included as a major or minor active ingredient in a composition for preventing or inhibiting fibrosis in cells.
[10] On the other hand, another aspect of the present disclosure relates to a method of preventing or inhibiting fibrosis in a cell by treating the cell with a composition including hyaluronan and proteoglycan link protein 1 (HAPLN1) or the gene encoding HAPLN1 as an active ingredient.
[11] Furthermore, an additional aspect of the present disclosure relates to a kit for preventing or inhibiting fibrosis in cells, including a composition according to any one of items [1] to item [9] and instructions for treatment according to the method of item [10].
[12] Furthermore, an additional aspect of the present disclosure relates to an experimental reagent composition relating to the prevention or inhibition of fibrosis in cells, including a composition according to any one of items [1] to item [9].

### Advantageous Effects

As described above, the compositions and methods of the present disclosure prevent and inhibit cellular fibrosis, thereby fundamentally inhibiting the development or progression of various diseases in which cellular fibrosis is involved, thereby dramatically increasing the potential for preventing or treating such diseases. In other words, it is possible to prevent or treat fibrosis that occurs in each tissue due to a decrease in tissue regeneration and repair ability caused by environmental factors such as aging and smoking.

The compositions of the present disclosure have fewer side effects than existing drugs for treating fibrotic diseases and may safely and effectively cope with fibrotic diseases that are increasing every year along with the global aging trend.

The present disclosure also provides methods of preventing or treating diseases caused by fibrosis by using the compositions of the present disclosure.

In addition, the reagent compositions of the present disclosure may be used in the study of various diseases associated with cellular fibrosis to help identify the mechanisms of such diseases and to develop compositions for the prevention or treatment of such diseases.

### Description of Drawings

FIG. 1A is a photograph of protein bands from a western blot, indicating the anti-fibrotic action of the compositions of the present disclosure on skin fibrosis, using a normal human dermal fibroblast (NHDF) as a sample. The expression of αSMA and GAPDH proteins in the presence of the compositions of the present disclosure and various concentrations of pirfenidone are shown as intensities of a western blot band.
FIG. 1B is a graph showing the relative levels of αSMA in response to various concentrations of the compositions of the present disclosure and pirfenidone, quantifying the intensity of a western blot band of FIG. 1A.
FIG. 2A is a diagram of an experimental scheme to evaluate the anti-fibrotic action of the composition of the present disclosure on liver fibrosis using a human hepatic stellate cell (HHSC) as a sample.
FIG. 2B is a photograph of a western blot band representing the expression of αSMA and GAPDH proteins in response to various concentrations of the composition of the present disclosure and pirfenidone according to the experimental scheme of FIG. 2A.
FIG. 2C is a graph quantifying the intensity of a western blot band of FIG. 2A, indicating the relative levels of αSMA in response to various concentrations of the compositions of the present disclosure and pirfenidone.
FIG. 3A is an experimental scheme for evaluating the anti-fibrotic action of the compositions of the present disclosure on intestinal fibrosis using a human colon fibroblast cell line (CCD-18Co) as a sample.
FIG. 3B is a photograph of a western blot band representing the expression of αSMA and GAPDH proteins in response to various concentrations of the composition of the present disclosure and pirfenidone according to the experimental scheme of FIG. 3A.
FIG. 3C is a graph quantifying the intensity of a western blot band of FIG. 3B, indicating the relative levels of αSMA in response to various concentrations of the compositions of the present disclosure and pirfenidone.
FIG. 4A is an experimental scheme for evaluating the anti-fibrotic action of the compositions of the present disclosure on cardiac fibrosis using a human cardiac microvascular endothelial cell (HCMEC) as a sample.
FIG. 4B is a photograph of a western blot band representing the expression of αSMA and GAPDH proteins in response to various concentrations of the composition of the present disclosure and pirfenidone according to the experimental scheme of FIG. 4A.
FIG. 4C is a graph showing the relative levels of αSMA in response to various concentrations of the compositions of the present disclosure and pirfenidone by quantifying the intensity of a western blot band of FIG. 4B.
FIG. 5A is a photograph of a western blot band showing the anti-fibrotic action of the compositions of the present disclosure on skin fibrosis using a normal human lung fibroblast (NHLF) as a sample. The expression of αSMA and GAPDH proteins in response to various concentrations of the composition of the present disclosure and pirfenidone is shown as the intensity of a western blot band.
FIG. 5B is a graph showing the relative levels of αSMA in response to various concentrations of the compositions of the present disclosure and pirfenidone by quantifying the intensity of a western blot band of FIG. 5A.
FIG. 6A is an experimental scheme for evaluating the fibrosis prevention action of the compositions of the present disclosure on kidney fibrosis using the epithelial cell line of human kidney renal tubule cell (Human Kidney 2, HK-2) as a sample.
FIG. 6B is a photograph of a western blot band representing the expression of αSMA and GAPDH proteins in response to various concentrations of the composition of the present disclosure and pirfenidone according to the experimental scheme of FIG. 6A.
FIG. 6C is a graph quantifying the intensity of a western blot band of FIG. 6B, indicating the relative levels of αSMA in response to various concentrations of the compositions of the present disclosure and pirfenidone.
FIG. 7A is an experimental scheme for evaluating the anti-fibrotic action of the compositions of the present disclosure on kidney fibrosis using the epithelial cell line of human kidney renal tubule cell (Human Kidney 2, HK-2) as a sample.
FIG. 7B is a photograph of a western blot band representing the expression of αSMA and GAPDH proteins in response to various concentrations of the composition of the present disclosure and pirfenidone according to the experimental scheme of FIG. 7A.
FIG. 7C is a graph quantifying the intensity of a western blot band of FIG. 7B, indicating the relative levels of αSMA in response to various concentrations of the compositions of the present disclosure and pirfenidone.
FIG. 8A is an experimental scheme for evaluating the efficacy of rhHAPLN1 in altering TGFβ1-induced fibrotic morphology of the HK-2 cell, using the epithelial cell line of human kidney renal tubule cell (Human Kidney 2, HK-2) as a sample.
FIG. 8B is a micrograph illustrating the morphological changes of cells in response to various concentrations of the compositions of the present disclosure and pirfenidone according to the experimental scheme of FIG. 8A.
FIG. 9A is a photograph of a western blot band showing the anti-fibrotic action of the composition of the present disclosure against age-induced fibrosis using a renal proximal tubule epithelial cell (RPTEC) as a sample. The expression of αSMA and GAPDH proteins in response to different concentrations of the composition of the present disclosure on an 11th passage cultured cell is shown as the intensity of a western blot band.
FIG. 9B is a graph showing the relative levels of αSMA in response to various concentrations of the compositions of the present disclosure by quantifying the intensity of a western blot band presented in FIG. 9A.
FIG. 10A is a photograph showing the nuclei of cells stained with DAPI (4',6-diamidino-2-phenylindole) at a 4th passage culture and an 11th passage culture, and the fluorescence of αSMA antibody (abeam, ab7817) diluted 1:1000 in 1XPBST (1X PBS with 0.1 % Triton X-100, 1 % BSA) to stain intracellular αSMA.
FIG. 10B is a fluorescence photograph showing the relative level of αSMA in cells according to various concentrations of the composition of the present disclosure and pirfenidone treatment in a 4th passage culture as a control and in an 11th passage culture with induced senescence.
FIG. 11A is a graph showing the significant reduction effect of αSMA and collagen I by the composition of the present disclosure (rhHAPLN1) at different concentrations in the SAEMyoF system, a pulmonary fibrosis disease model, as a fold change compared to the vehicle control, in an experiment to confirm the effect of rhHAPLN1 on anti-fibrotic markers using the BioMAP Fibrosis panel.
FIG. 11B is a graph showing the significant reduction effect of the composition of the present disclosure (rhHAPLN1) on collagen I in the REMyoF system, a kidney fibrosis disease model, by concentration as a relative fold change compared to the vehicle control, as an experiment to confirm the effect of rhHAPLN1 on anti-fibrotic markers using the BioMAP Fibrosis panel.
FIG. 11C is a graph showing the significant reduction effect of myofibroblast to the composition of the present disclosure (rhHAPLN1) on collagen IV by concentration as a relative multiple compared to the vehicle control, in an experiment confirming the effect of rhHAPLN1 on anti-fibrotic markers using the BioMAP Fibrosis panel.
FIG. 12A is a diagram outlining an experiment to evaluate the anti-fibrotic efficacy (prevention of fibrotic disease) of the composition of the present disclosure using a mouse bleomycin-induced pulmonary fibrosis (BIPF) model.

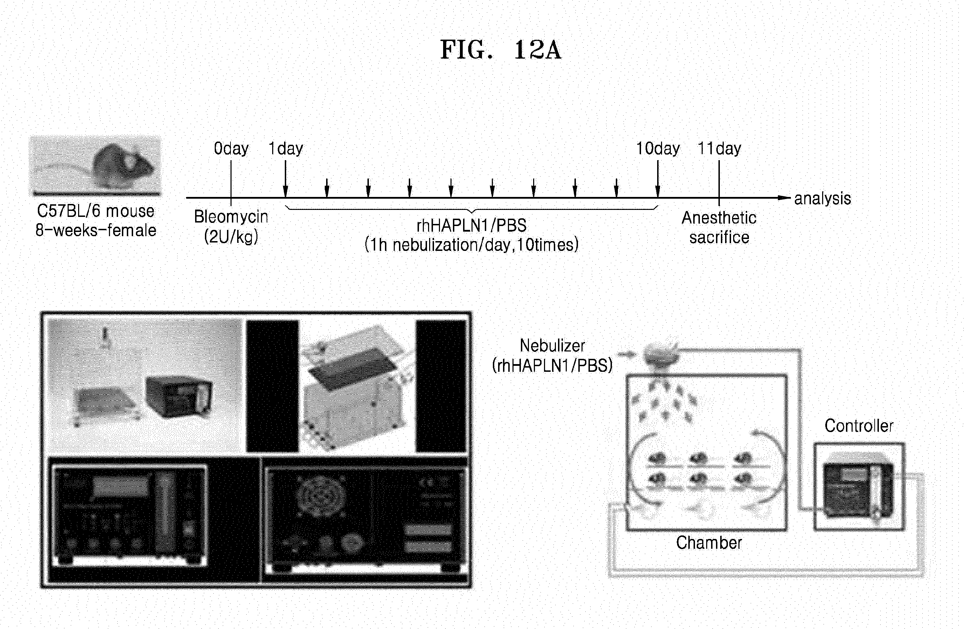

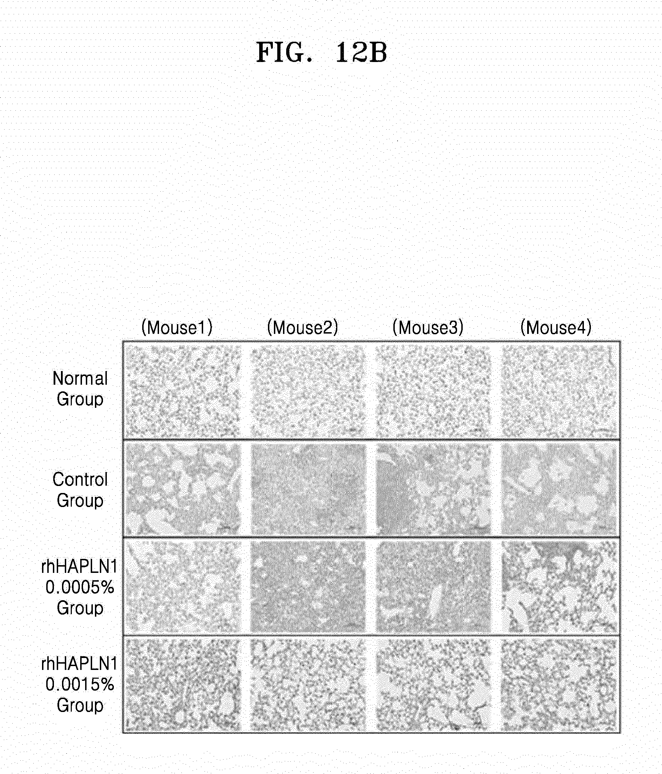

FIG. 12B is a photograph of an experiment using the mouse bleomycin-induced pulmonary fibrosis model of FIG. 12A, in which mice were divided into four groups (Mouse 1 to 4), wherein the Normal group was treated with PBS, the control was not treated, and the composition of the present disclosure (rhHAPLN1) 0. 0005 % (w/w), and 0.0015 % (w/w) of the composition of the present disclosure (rhHAPLN1), respectively, and the lung tissue of four mice per group was harvested, and the left large lobe was cut in half transversely, and the upper portion was fixed by placing it in formalin, and hematoxylin and eosin (H&E) staining was performed.
FIG. 12C is a graph in which three tissue slides were made per mouse by cutting the middle portion of the lung tissue of the largest lobe of the left side of the mouse, and three sections were randomly photographed on each slide, and then the stained darker part and the unstained white part were distinguished for a total of nine sections, and the area of the stained darker part was measured using "Image J" software, and each figure obtained was averaged to obtain an average value per mouse to confirm statistical significance.
FIG. 12D and FIG. 12E are photographs and descriptions, respectively, of the Ashcroft score, which provides a formal guideline for a simple visual measurement of the severity of pulmonary fibrosis (Ashcroft et al 1988, J Clin Pathol 41:467-470).
FIG. 12F is a graph of the results of an experiment according to FIG. 13A and FIG. 13B in which the severity of pulmonary fibrosis is represented by the Ashcroft score.
FIG. 13A is a diagram outlining an experiment to evaluate the anti-fibrotic efficacy (ability to treat fibrotic disease) of the compositions of the present disclosure using a mouse bleomycin-induced pulmonary fibrosis (BIPF) model.
FIG. 13B is a photograph of an experiment using the mouse bleomycin-induced pulmonary fibrosis model of FIG. 13A, in which mice were divided into four groups (Normal, PBS Control, 0.00075 % (w/w) rhHAPLN1, 0.0015 % (w/w) rhHAPLN1, 0. 003 %(w/w) rhHAPLN1) and set at 4 mice per group, after the experiment, the lung tissue of 4 mice per group was harvested, and the left large lobe was cut in half transversely, and the upper portion was fixed by placing it in formalin, and hematoxylin and eosin (H&E) staining was performed.
FIG. 13C and FIG. 13D are graphs in which three tissue slides were made per mouse by cutting the middle portion of the lung tissue of the largest lobe of the left side of the mouse, and three sections were photographed randomly on each slide, and then the stained darker part and the unstained white part were distinguished for a total of nine sections, and each figure obtained by measuring the area of the stained darker part using "Image J" software was averaged to obtain an average value per mouse to confirm statistical significance.
FIG. 13E is a graph of the results of an experiment expressing the severity of pulmonary fibrosis as Ashcroft score in relation to the results of FIG. 13C.
FIG. 14A is a diagram of an experimental scheme to evaluate the anti-fibrotic action of the composition of the present disclosure on kidney fibrosis using ischemia/reperfusion as an animal model of kidney fibrosis induction.
FIG. 14B is a photograph of protein bands from a western blot indicating the degree of expression of αSMA protein following administration of the composition of the present disclosure and pirfenidone according to the experimental scheme of FIG. 14A (SV; sham-vehicle, SB; sham-rhHAPLN1 dose B, and a group to confirm the effect of treating the sham control with rhHAPLN1, IRV; IR-vehicle, IRP; IR-pirfenidone, IRA; IR-rhHAPLN1 dose A, IRB; IR-rhHAPLN1 dose B, IRC; IR-rhHAPLN1 dose C).
FIG. 14C is a graph showing the fold change in the expression level of αSMA against IRV in response to various concentrations of pirfenidone and compositions of the present disclosure, quantifying the intensity of a western blot band of FIG. 14B.
FIG. 14D is a photograph of Sirus red and PAS staining showing the expression level of collagen in response to various concentrations of pirfenidone and the composition of the present disclosure in an acute kidney injury induced model. Purple color shows collagen expression.
FIG. 14E is a photograph showing only collagen-positive areas after imaging two random sites in the outer medulla region of the kidney in tissue staining to quantify the expression of collagen in response to various concentrations of pirfenidone and the composition of the present disclosure in an acute kidney injury induced model.
FIG. 14F is a graph showing the marking of collagen-positive areas in FIG. 14E and the quantification of the area (%) of collagen using i-solution software (IMT).
FIG. 14G is a graph showing creatinine clearance at various concentrations of pirfenidone and the compositions of the present disclosure in an acute kidney injury induced model, measured at day 21 after ischemia/reperfusion.

### Best Mode

First, various terms appearing in this specification may be defined as follows.

As used herein, "rhHAPLN1" is an abbreviation for recombinant human HAPLN1 and represent recombinant human HAPLN1.

As used herein, "fibroblast" refers to a cell involved in the maintenance of tissue structure by producing connective tissue, such as collagen fibers, other than epithelial, vascular, lymphatic, and inflammatory cells.

As used herein, "myofibroblast" refers to a fibroblast that is activated by physical injury or inflammation, etc. and expresses α-SMA (alpha-smooth muscle actin) and has a contractile function like a smooth muscle cell.

As used herein, "αSMA" or "α-SMA (alpha smooth muscle actin)" is a protein expressed in a pathological vascular smooth muscle cell and a stromal fibroblastic cell, and is a major marker of tissue fibrosis. Expression is high in kidneys with evidence of kidney fibrosis, and this high expression or activity indicates high myofibroblast activity, which is closely related to the degree of interstitial fibrosis. Furthermore, the expression of αSMA in patients is closely associated with a decrease in renal creatine clearance, and thus serves as a marker for the inability of the body to clear creatine as a waste product.

As used herein, "transforming growth factor beta 1 (TGFβ1 or TGF-β1)" refers to a cytokine that is used in various examples of the present disclosure to transform fibroblasts into myofibroblasts, with varying amounts of conversion capacity per cell. In general, the transformation of fibroblasts into a myofibroblasts involve a variety of bioactive substances, including cytokines, chemokines, growth factors, and hormones, etc.

As used herein, pirfenidone is an anti-fibrotic drug that has been approved for the treatment of pulmonary fibrosis and is in clinical trials for the treatment of kidney fibrosis. Pirfenidone was used as a control substance in the experiments of the examples of the present disclosure. Effective doses may vary from cell to cell, but have been reported in related literature to be effective from 100 µg/mL or more.

Hereinafter, the present disclosure will be described in detail.

One aspect of the present disclosure relates to an anti-fibrotic composition including as an active ingredient hyaluronan and proteoglycan link protein 1 (HAPLN1) or a gene encoding the same. The composition may function as a pharmaceutical composition for the prevention or treatment of fibrotic diseases.

Furthermore, according to an embodiment of the present disclosure, the composition of the present disclosure may be a recombinant human HAPLN1 protein represented by SEQ ID NO: 1. In this regard, the HAPLN1 protein of the present disclosure may be a protein that has 80 %, preferably 85 %, more preferably 90 %, still more preferably 95 %, and most preferably 100 % sequence identity to the amino acid sequence of SEQ ID NO: 1, as long as the protein retains its anti-fibrotic function.

Furthermore, according to an embodiment of the present disclosure, the nucleic acid for the gene encoding the HAPLN1 protein in the composition of the present disclosure may be included in the composition by being included in an expression vector.

Furthermore, in the present disclosure, the cells on which the compositions of the present disclosure act are not particularly limited, but preferably include various fibroblasts, astrocytes, endothelial cells, and epithelial cells, etc., and more specifically include skin fibroblasts, hepatic stellate cells, human colon cells, microvascular endothelial cells, lung fibroblasts, kidney renal tubule cells, and proximal tubule epithelial cells, etc., and include cells in various situations such as in vivo, in vitro, ex vivo, and in situ, etc. Furthermore, the applied organ is not particularly limited as long as it is an organ that is likely to cause fibrosis, but preferably includes lung, kidney, skin, liver, intestine, heart, etc.

Furthermore, according to an embodiment of the present disclosure, the composition of the present disclosure is administered to the cells in a single dose of 0.1 ng/ml to 500 ng/ml, preferably 1 ng/ml to 300 ng/ml, more preferably 3.0 ng/ml to 50 ng/ml, and even more preferably 10 ng/ml to 13 ng/ml. However, the dosage may be 3 ng/ml, 5 ng/ml, 11 ng/ml, 30 ng/ml, 50 ng/ml, or 100 ng/ml, depending on the area of application, or more specifically as the case may be.

Alternatively, according to an embodiment of the present disclosure, the composition of the present disclosure is administered to the cells in a single dose of 0.00001 % (w/w) to 0.1 % (w/w), preferably 0.0001 % (w/w) to 0.05 % (w/w), more preferably 0.0003 % (w/w) to 0.03 % (w/w), and even more preferably 0.0005 % (w/w) to 0.015 % (w/w).

On the other hand, in an embodiment of the present disclosure, the composition of the present disclosure may be included as a major or minor active ingredient in a composition for preventing or inhibiting fibrosis of cells.

Furthermore, according to an aspect of the present disclosure, the aspect relates to a method of preventing or inhibiting fibrosis of cells by treating cells with a composition including as an active ingredient hyaluronan and proteoglycan link protein 1 (HAPLN1) or the gene encoding HAPLN1.

In addition, according to an aspect of the present disclosure, the aspect relates to a kit for preventing or inhibiting fibrosis of cells, including the composition of the present disclosure and processing instructions.

In addition, according to an aspect of the present disclosure, the aspect relates to a reagent composition for experiments related to preventing or inhibiting fibrosis of cells, including the composition of the present disclosure.

Further, the present disclosure relates to the use of a composition of the present disclosure for use in the preparation of a pharmaceutical composition for the prevention or treatment of a disease caused by fibrosis of cells.

In a certain embodiment, the pharmaceutical composition may generally include a molecule and a pharmaceutically acceptable carrier. As used herein, the term "pharmaceutically acceptable carrier" include saline solution, solvent, dispersion media, coating, antibacterial and antifungal agent, isotonic agent, and absorption retarder, etc. that are compatible with pharmaceutical administration. Supplementary active compounds may also be included in the composition. In other words, the pharmaceutical composition of the present disclosure may further include pharmaceutical additives selected from the group consisting of a pharmaceutically acceptable carrier, diluent, binder, disintegrating agent, lubricant, and any combination thereof.

The pharmaceutical composition may be formulated to be compatible with the intended route of administration. Preferably, the composition of the present disclosure may be in a dosage form selected from the group consisting of an eye drop, ointment, tablet, pill, capsule, troche, inhalant, injection, patch, and suppository.

Examples of routes of administration include parenteral, for example, intravenous, intradermal, subcutaneous, oral (for example, inhalation), transdermal (topical), transmucosal, and rectal administration. Preferably for parenteral administration.

Solutions or suspensions used for parenteral, intradermal or subcutaneous application may include the following ingredients: sterile diluents such as injectable water, saline, fixative oil, polyethylene glycol, glycerin, propylene glycol, or other synthetic solvents; antibacterial agents such as benzyl alcohol or methyl paraben; antioxidants such as ascorbic acid or sodium bisulfite; chelating agents such as ethylenediaminetetraacetic acid; buffers such as acetate, citrate or phosphate and tonicity-adjusting agents such as sodium chloride or dextrose. The pH may be adjusted with an acid or base, such as hydrochloric acid or sodium hydroxide. The parenteral preparation may be in an ampoule, disposable syringe, or multi-dose vial made of glass or plastic.

The pharmaceutical composition suitable for injection include sterile aqueous solution (if water soluble) or dispersion and sterile powder for the extemporaneous preparation of sterile injectable solution or dispersion. Carriers suitable for intravenous administration include physiological saline, bacteriostatic water, or phosphate buffered saline (PBS). In all cases, the composition should be sterile and fluid enough to facilitate injection. The composition be stable under manufacturing and storage conditions and preserved against the contaminating action of microorganisms such as bacteria and fungi. The carrier may be a solvent or dispersion medium containing, for example, water, ethanol, polyols (for example, glycerol, propylene glycol, and liquid polyethylene glycol), and suitable mixtures thereof. For example, appropriate fluidity may be maintained by the use of a coating such as lecithin, maintenance of the required particle size in case of dispersion, and use of a surfactant. Prevention of microbial action may be achieved by various antibacterial and antifungal agents, for example, paraben, chlorobutanol, phenol, ascorbic acid, thimerosal, etc. In many cases it will be desirable to include in the composition an isotonic agent, for example a sugar, a polyalcohol such as mannitol, sorbitol, sodium chloride. Prolonged absorption of the injectable composition may be achieved by including in the composition an agent that delay absorption, such as aluminum monostearate and gelatin.

Sterile injectable solutions may be prepared by mixing the required amount of the active compound in a suitable solvent with one or a combination of the ingredients listed above as required, followed by filtration sterilization. Generally, dispersions are prepared by incorporating the active compound into a sterile vehicle including a base dispersion medium and the other necessary ingredients listed above. For sterile powders for the preparation of sterile injection solutions, preferred methods of preparation are vacuum drying and lyophilization, which produce a powder of the active ingredient and any desired additional ingredients from a previously sterile filtered solution.

Oral compositions typically include an inert diluent or an edible carrier. For the purpose of oral therapeutic administration, the active compound may be mixed with excipients and may be used in the form of a tablet, troche or capsule, for example a gelatin capsule. The oral composition may also be prepared using a fluid carrier for use as a mouthwash. Pharmaceutically suitable binder and/or excipient materials may be included as part of the composition. Tablets, pills, capsules, troches, etc. may contain any of the following components or compounds of similar nature : a binder, such as microcrystalline cellulose, tragacanth gum, or gelatin; an excipient, such as starch or lactose; a disintegrant, such as alginate, primogel, or cornstarch; a lubricant, such as magnesium stearate or sterotes; a lubricant, such as colloidal silicon dioxide; a sweetener, such as sucrose or saccharin; or a flavor, such as peppermint, methyl salicylate, or orange flavor.

For administration by inhalation, the compound is delivered in the form of an aerosol spray from a pressure vessel or dispenser containing a suitable propellant, for example, a gas such as carbon dioxide, or a nebulizer.

Systemic administration may also be accomplished by transmucosal or transdermal means. For transmucosal or transdermal administration, a penetrant suitable for the barrier to be penetrated is used in the formulation. Such penetrants are generally known in the art and include, for example, a detergent, bile salt, and fusidic acid derivative for transmucosal administration. Transmucosal administration may be accomplished using a nasal spray or suppository. For transdermal administration, the active compound is formulated as an ointment, plaster, gel, or cream as is generally known in the art.

Compounds may also be formulated in the form of a suppository (for example, with usual suppository bases such as cocoa butter and other glycerides) or retention enemas for rectal delivery.

Data obtained from cell culture assays and animal studies may be used to formulate various doses for use in humans. The dosage of these compounds is preferably within a range of circulating concentrations that include the ED₅₀ with little or no toxicity. Dosages may vary within this range depending on the dosage form used and the route of administration used.

The therapeutically effective amount (in other words, effective dose) of a composition of the present disclosure will depend on the circumstances of the selected patient. For example, a single dose in the range of about 1 pg to 1,000 mg may be administered; in some embodiments, 10 pg, 30 pg, 100 pg, or 1,000 pg, or 10 ng, 30 ng, 100 ng, or 1,000 ng, or 10 µg, 30 µg, 100 µg, or 1,000 µg, or 10 mg, 30 mg, 100 mg, or 1,000 mg may be administered.

Furthermore, according to an embodiment of the present disclosure, when the composition of the present disclosure is administered directly to a subject in vivo, the dose of rhHAPLN1 protein is 0.001 mg/kg/BW to 10 mg/kg/BW, preferably 0.004 mg/kg/BW to 5 mg/kg/BW, more preferably 0.1 mg/kg/BW to 1.0 mg/kg/BW, even more preferably 0.15 mg/kg/BW to 0.5 mg/kg/BW.

Alternatively, in some embodiments, a pharmaceutical composition of 1 ng/ml to 100 µg/ml, preferably 3 ng/ml to 50 µg/ml, more preferably 5 ng/ml to 500 ng/ml, particularly preferably 10 ng/ml to 200 ng/ml may be administered. The pharmaceutical composition may be administered from at least once daily to at least once weekly, including once every other day. The skilled technician will recognize that certain factors may affect the dosage and timing required to effectively treat an individual, including, but not limited to, the severity of the disease or disorder, prior treatment, the general health and/or age of the individual, and other pre-existing conditions. Furthermore, treating an individual with a therapeutically effective amount of a molecule of the present disclosure may include a single treatment, or preferably a series of treatments.

The compositions of the present disclosure may be administered at doses ranging from 5 mg/kg/week to 500 mg/kg/week, for example, 5 mg/kg/week, 10 mg/kg/week, 15 mg/kg/week, 20 mg/kg/week, 25 mg/kg/week, 30 mg/kg/week, 35 mg/kg/week, 40 mg/kg/week, 45 mg/kg/week, 50 mg/kg/week, 55 mg/kg/week, 60 mg/kg/week, 65 mg/kg/week, 70 mg/kg/week, 75 mg/kg/week, 80 mg/kg/week, 85 mg/kg/week, 90 mg/kg/week, 95 mg/kg/week, 100 mg/kg/week, 150 mg/kg/week, 200 mg/kg/week, 250 mg/kg/week, 300 mg/kg/week, 350 mg/kg/week, 400 mg/kg/week, 450 mg/kg/week, and 500 mg/kg/week, depending on the patient's situation. In a certain embodiment, the dosage of a bifunctional molecule according to the present disclosure is in the range of 10 mg/kg/week to 200 mg/kg/week, 20 mg/kg/week to 150 mg/kg/week, or 25 mg/kg/week to 100 mg/kg/week. In a certain embodiment, the composition of the present disclosure is administered 1x per week for a period of 2 weeks to 6 months, for example, 2 weeks, 3 weeks, 4 weeks, 5 weeks, 6 weeks, 7 weeks, 8 weeks, 9 weeks, 10 weeks, 11 weeks, 12 weeks, 13 weeks, 26 weeks, 6 months, 8 months, 10 months, or 1 year or more. In a certain embodiment, the compositions of the present disclosure is administered 2x per week. In another embodiment, the compositions of the present disclosure is administered biweekly.

The compositions of the present disclosure may also be formulated into pharmaceutical compositions including a pharmaceutically effective amount of a HAPLN1 protein molecule and a pharmaceutically acceptable carrier. A pharmaceutically or therapeutically effective amount refers to a content that is effective to produce an intended pharmacologic, therapeutic, or prophylactic result. The phrases "pharmacologically effective amount" and "therapeutically effective amount" or simply "effective amount" refer to the amount of a bifunctional molecule that is effective to produce an intended pharmacological, therapeutic, or prophylactic result. For example, if a given clinical treatment is considered effective when a measurable parameter associated with a disease or disorder is reduced by at least 20 %, then the therapeutically effective amount of a drug for treating that disease or disorder is the amount necessary to reduce that parameter by at least 20 %.

Suitably formulated pharmaceutical compositions of the present disclosure may be administered by any means known in the art, such as intravenous, intramuscular, intraperitoneal, subcutaneous, transdermal, airway (aerosol), rectal, vaginal, and parenteral routes, including topical (including buccal and sublingual) administration. In some embodiments, the pharmaceutical composition is administered by intravenous or parenteral infusion or injection.

In general, suitable dosage units of a molecule are in the range of 0.001 mg to 0.25 mg per kg of body weight of the recipient per day, or in the range of 0.01 µg to 20 µg per kg of body weight per day, or in the range of 0.01 µg to 10 µg per kg of body weight per day, or in the range of 0.10 µg to 5 µg per kg of body weight per day, or in the range of 0.1 µg to 2.5 µg per kg of body weight per day. The pharmaceutical composition including the molecule may be administered once daily. However, the therapeutic agent may also be administered in dosage units including 2, 3, 4, 5, 6 or more sub-doses administered at appropriate intervals throughout the day. The dosage unit may also be formulated as a single dose over several days, for example, using a usual sustained release formulation that provide a sustained and consistent release of the molecule over a period of several days. Sustained release formulations are well known in the art. In this embodiment, the dosage unit includes multiples corresponding to the daily dosage.

The pharmaceutical composition may be included in a kit, container, pack, or dispenser with instructions for administration.

As used herein, "treat" or "treating" is defined as the application or administration of a therapeutic agent (for example, a molecule of the present disclosure) to a patient, or the application or administration of a therapeutic agent to an isolated tissue or cell line, for the purpose of treating, curing, alleviating, mitigating, modifying, relieving, improving, ameliorating or affecting a disease or disorder, a symptom of a disease or disorder, or a predisposition to a disease or disorder, wherein the patient has a disease or disorder, a symptom of a disease or disorder, or a predisposition to a disease or disorder.

Furthermore, an aspect of the present disclosure relates to the use of a composition including hyaluronan and proteoglycan link protein 1 (HAPLN1) as an active ingredient in preventing or inhibiting fibrosis in cells.

### Mode for Invention

### Example

The present disclosure will be described in more detail through the following examples, but the following Examples are for the purpose of illustration only and are not intended to limit the scope of the present disclosure.

### [Preparation Example 1]

### Preparation of hyaluronan and proteoglycan link protein 1 (HAPLN1).

A CHO-K1 cell line producing recombinant human HAPLN1 protein was constructed by inserting a vector including a polynucleotide encoding human HAPLN1 protein of amino acid SEQ ID NO: 1 into CHO-K1 cells.

The amino acid SEQ ID NO: 1 is as follows:

MKSLLLLVLISICWADHLSDNYTLDHDRAIHIQAENGPHLLVEAEQAKVFSHRG
GNVTLPCKFYRDPTAFGSGIHKIRIKWTKLTSDYLKEVDVFVSMGYHKKTYGGYQGRV
FLKGGSDSDASLVITDLTLEDYGRYKCEVIEGLEDDTVVVALDLQGVVFPYFPRLGRYN
LNFHEAQQACLDQDAVIASFDQLYDAWRGGLDWCNAGWLSDGSVQYPITKPREPCG
GQNTVPGVRNYGFWDKDKSRYDVFCFTSNFNGRFYYLIHPTKLTYDEAVQACLNDGA
QIAKVGQIFAAWKILGYDRCDAGWLADGSVRYPISRPRRRCSPTEAAVRFVGFPDKKH
KLYGVYCFRAYN

Cell lines with excellent protein production and quality were selected as a master cell bank (MCB). The MCB was passage cultured and inoculated into Thermo's Hyperforma SUB 250 L bioreactor at a concentration of 0.40±0.05×10⁶ cells/mL and fed-batch cultured. The basic medium used was 22.36 g ActiPro^{™} medium + 0.5846 g glutamine + 10.00 g HT Supplement (Thermo Fisher Scientific) + 4.29 g 10 N NaOH + 1.80 g NaHCOs. The culture temperature was set at 36.5 °C, dissolved oxygen (DO) was set at 40.0 %, and pH was set at 7.00±0.20. 1 M Sodium Carbonate Monohydrate was used as a pH adjustment solution. As feeding medium (FM), 181.04 g HyClone^{™} Cell Boost 7a + 12.28 g 10 N NaOH in FM020a and 94.60 g HyClone^{™} Cell Boost 7b + 105.93 g 10 N NaOH in FM020b were used.

Recombinant human HAPLN1 (rhHAPLN1) protein was isolated and purified from cells cultured as above through a process such as harvest and clarification, ultrafiltration/diafiltration 1 (UF/DF1), anion exchange chromatography, solvent/detergent (S/D) virus inactivation, cation exchange chromatography, mixed-mode chromatography (MMC), hydrophobic interaction chromatography, ultrafiltration/diafiltration 2 (UF/DF2) and intermediate depth filtration (Int. DF), etc. The following embodiments utilize these compositions of the present disclosure.

### [Preparation Example 2]

### Preparation of composition of present disclosure including HAPLN1 Protein

The HAPLN1 protein purified in Preparation Example 1 was stabilized in 20 mM acetic acid buffer, 8 % (w/v) sucrose, 0.04 % (w/v) PS80, pH 5.0 to prepare a composition of the present disclosure including HAPLN1 protein itself as the main active ingredient.

### [Preparation Example 3]

### Preparation of composition of present disclosure including expression vector carrying gene encoding HAPLN1 protein

A composition containing an expression vector containing a gene encoding the HAPLN1 protein was prepared using the jetPRIME Transfection Reagent Kit (PolyPlus, 114-15). More specifically, a plasmid vector designed to express Human HAPLN1 was prepared using OriGene product RC209274 (hereinafter referred to as "Human HAPLN1 ORF Clone"). This allows the vector to express the gene in transport within the nucleus of the host cell.

For reference, a jetPRIME transfection reagent kit consists of a jetPRIME buffer and jetPRIME transfection reagent, the jetPRIME buffer is used to dilute the plasmid vector to be transported into the host cell, and the jetPRIME transfection reagent is used to capture the plasmid vector in the form of a vesicle (liposome) for penetration into the host cell.

The Human HAPLN1 ORF Clone was diluted in jetPRIME buffer to prepare a composition of the present disclosure containing an expression vector containing the gene encoding the HAPLN1 protein.

In response, jetPRIME transfection reagent is added and left for 10 minutes to capture the plasmid vector in the form of a vesicle (liposome) and then dropped onto the cells in culture. After culturing the cells for at least 48 hours, the increase in human HAPLN1 gene expression was confirmed by real-time qPCR, and the increase in human HAPLN1 protein expression was confirmed by western blot.

### [Example 1]

### Evaluation of anti-fibrotic efficacy of composition of present disclosure on human dermal fibroblast

Normal human dermal fibroblast (NHDF) was used as a sample to verify the anti-fibrotic action of the composition of the present disclosure on skin fibrosis.

### 1. Experimental method

(1) Human dermal fibroblasts (normal human dermal fibroblast, NHDF) were seeded (1.0 × 10⁵ cells/well) in a 6-well plate and incubated in FBM-2 (fibroblast growth medium-2, CC-3131, Lonza) medium for 24 hours in an incubator at 37 °C 5 % CO₂.
(2) After replacing with serum-free medium, the medium was incubated for 24 hours.
(3) In this regard, under conditions in which fibrosis was induced by treatment with TGFβ1 (10 ng/ml), each well of the plate was treated with the composition of the present disclosure based on Preparation Example 2 but with rhHAPLN1 protein content of 0, 3, 10, 30, and 100 ng/ml and 100 µg/mL and 200 µg/mL of pirfenidone (Selleckchem), a commercially available anti-fibrotic agent, respectively, and incubated for 24 hours.
(4) Afterwards, each sample was washed twice with phosphate buffered saline (PBS, pH 7.2), and then the expression level of αSMA, a marker for myofibroblasts, was confirmed by western blot.

### 2. Result

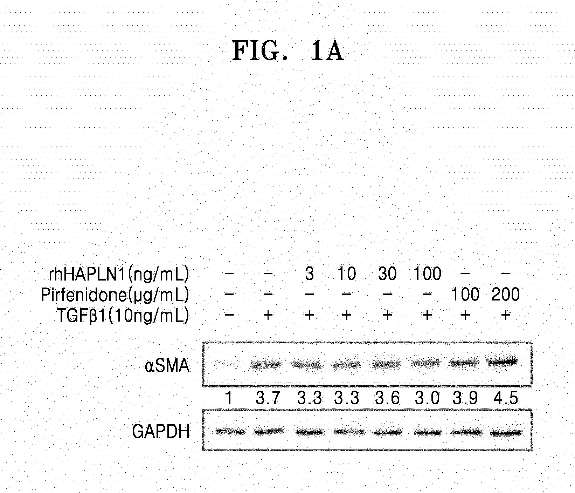

In FIG. 1A, it was confirmed from the western blot band photograph that the composition of the present disclosure exhibits a much better anti-fibrotic action than pirfenidone, judging from the intensity and thickness of the αSMA protein band in relation to the degree of fibrosis of normal human dermal fibroblasts.

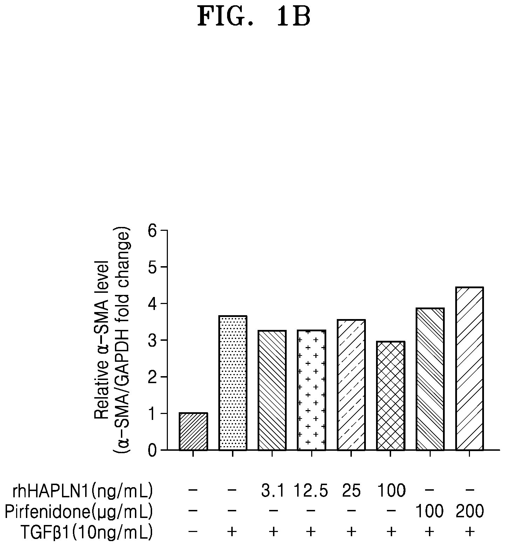

Furthermore, in FIG. 1B, after inducing fibrosis with TGF1β (10 ng/ml), the expression level of αSMA was significantly lower in all rhHAPLN1 protein content ranges when treated with the composition of the present disclosure compared to when treated with the composition that does not include rhHAPLN1 protein, indicating the anti-fibrotic efficacy. In particular, among the compositions of the present disclosure, the sample with rhHAPLN1 protein content of 100 ng/ml showed the most excellent anti-fibrotic efficacy, which was 25% higher in anti-fibrotic efficacy even though the content was 1/1,000th of that of pirfenidone at 100 µg/mL (100,000 ng/ml). Since 200 µg/mL of pirfenidone shows a higher expression level of αSMA than 100 µg/mL, the anti-fibrotic efficacy is lower at high concentrations, and it may be assumed that 100 µg/mL is the optimal anti-fibrotic concentration. Then, it was found that the composition of the present disclosure has much better anti-fibrotic efficacy than pirfenidone, a commercially available anti-fibrotic agent.

### [Example 2]

### Evaluation of anti-fibrotic efficacy of composition of present disclosure on human hepatic stellate cell

Human hepatic stellate cell (HHSC) was used as a sample to verify the anti-fibrotic action of the composition of the present disclosure on liver fibrosis.

### 1. Experimental method

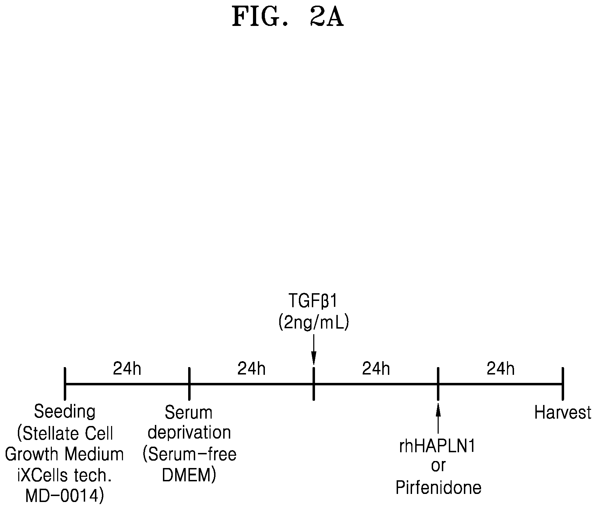

(1) Human hepatic stellate cells (primary human hepatic stellate cell (HHSC; iXCells Biotechnologies, 10HU-210) were seeded (1.5 × 10⁵ cells/well) in a 6-well plate according to the experimental scheme in FIG. 2A and incubated in stellate cell growth medium (iXCells Biotechnologies, MD-0014) in an incubator at 37 °C 5 % CO₂ for 24 hours.
(2) After replacing with serum-free DMEM medium, the medium was incubated for 24 hours.
(3) The plates were then treated with TGFβ1 (2 ng/ml) to induce fibrosis and incubated for 24 hours.
(4) Afterwards, each well of the plate was then treated with the composition of the present disclosure at rhHAPLN1 protein content of 0, 3, 10, 30, and 100 ng/ml and pirfenidone 100 µg/mL and 200 µg/mL, respectively, and incubated for 24 hours.
(5) Each sample was then treated with cell lysis buffer and harvested.
(6) Western blotting was performed on the samples. Here, anti-αSMA Ab (Abcam, ab7817), anti-GAPDH Ab (Santa Cruz, sc-32233) were used as primary antibodies and HRP-conjugated anti-mouse IgG Ab (CST, #7076) as secondary antibodies to confirm the protein expression levels of αSMA and GAPDH.

### 2. Result

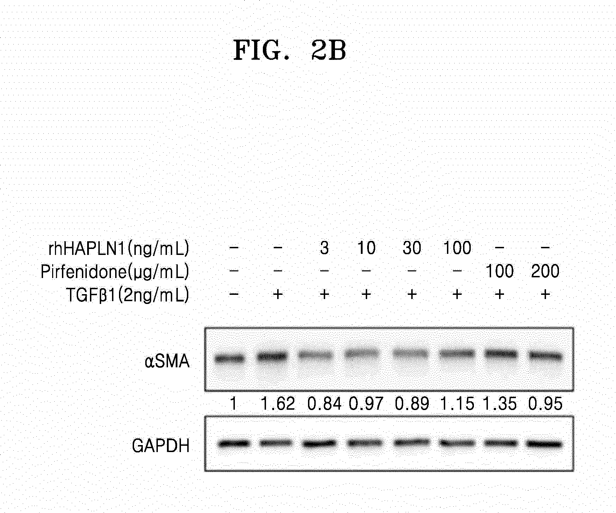

In FIG. 2B, it may be seen from the western blot band photographs that the composition of the present disclosure exhibits much better anti-fibrotic action compared to pirfenidone in all rhHAPLN1 protein content ranges, as indicated by the darkness and thickness of the αSMA protein band, in terms of the extent of fibrosis of human hepatic stellate cells.

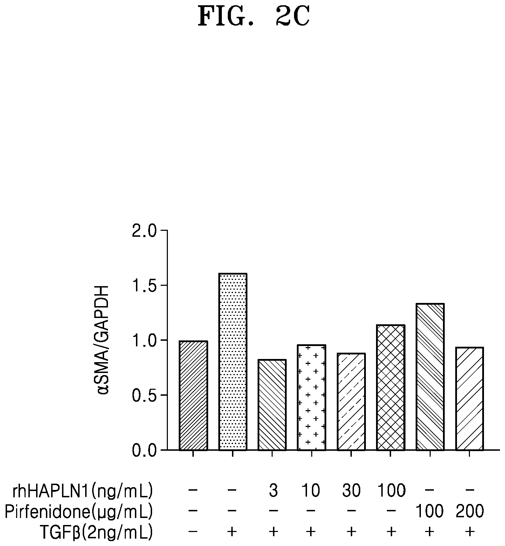

Furthermore, FIG. 2C shows that after inducing fibrosis with TGFβ1 (2 ng/ml), the expression level of αSMA as a marker of hepatic stellate cell activity was significantly lower in all rhHAPLN1 protein content ranges when treated with the composition of the present disclosure compared to when treated with the composition that does not include rhHAPLN1 protein, indicating the anti-fibrotic efficacy. In particular, among the compositions of the present disclosure, the sample with rhHAPLN1 protein content of 3 ng/ml showed the most excellent anti-fibrotic efficacy, which showed much higher anti-fibrotic efficacy even though the content was 3/200,000th of that of pirfenidone at 100 µg/mL (100,000 ng/ml) as well as the higher dose of pirfenidone at 200 µg/mL (200,000 ng/ml).

### [Example 3]

### Evaluation of anti-fibrotic efficacy of composition of present disclosure on human colon fibroblast cell line

Human colon fibroblast cell line (CCD-18Co) was used as a sample to verify the anti-fibrotic action of the composition of the present disclosure on intestinal fibrosis.

### 1. Experimental method

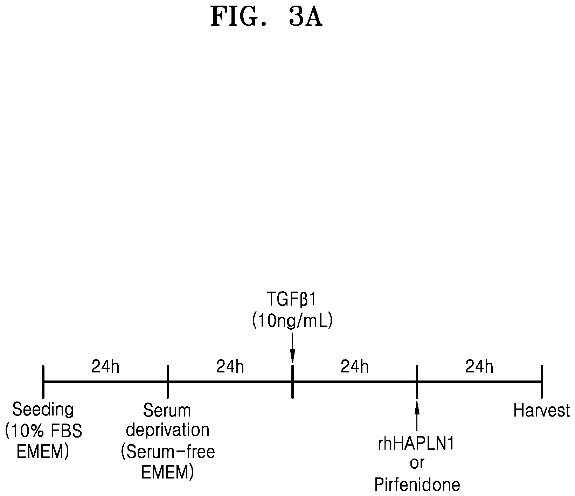

(1) A human colon fibroblast cell line (CCD-18Co (human colon fibroblast cell line; ATCC, CRL-1459)) was seeded (1.5 × 10⁵ cells/well) in a 6-well plate according to the experimental scheme in FIG. 3A and incubated in Eagle's minimum essential medium (EMEM) medium including 10 % fetal bovine serum (FBS) for 24 hours in an incubator at 37 °C and 5 % CO₂.
(2) After replacing with serum-free EMEM medium, the medium was incubated for 24 hours.
(3) The plates were then treated with TGF-β (10 ng/ml) to induce fibrosis and incubated for 24 hours.
(4) Afterwards, each well of the plate was then treated with the composition of the present disclosure at rhHAPLN1 protein content of 0, 3, 10, 30, and 100 ng/ml and pirfenidone 100 µg/mL and 200 µg/mL, respectively, and incubated for 24 hours.
(5) Each sample was then treated with cell lysis buffer and harvested.
(6) Western blot was performed on the samples. Here, anti-αSMA Ab (Abeam, ab7817), anti-GAPDH Ab (Santa Cruz, sc-32233) were used as primary antibodies and HRP-conjugated anti-mouse IgG Ab (CST, #7076) as secondary antibodies to confirm the protein expression levels of αSMA and GAPDH.

### 2. Result

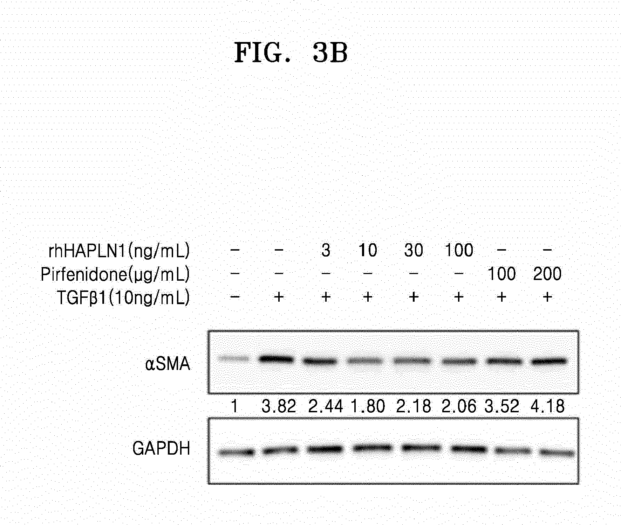

In FIG. 3B, it may be seen from the western blot band photographs that the composition of the present disclosure exhibits much better anti-fibrotic action compared to pirfenidone in all rhHAPLN1 protein content ranges, as indicated by the darkness and thickness of the αSMA protein band, in terms of the extent of fibrosis of human colon fibroblasts.

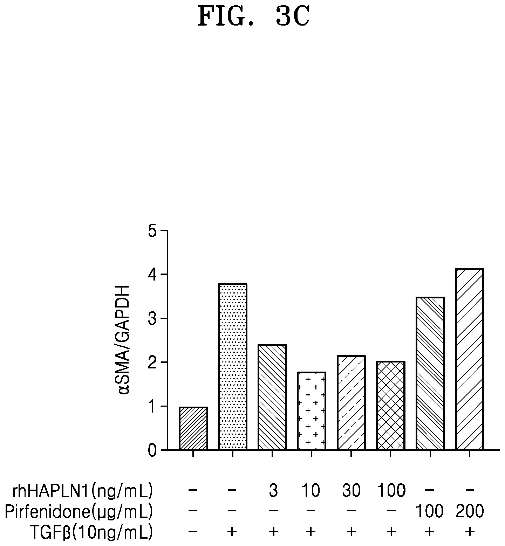

Furthermore, FIG. 3C shows that after inducing fibrosis with TGFβ1 (10 ng/ml), the expression level of αSMA as a myofibroblast marker was significantly lower in all rhHAPLN1 protein content ranges when treated with the composition of the present disclosure compared to when treated with the composition that does not include rhHAPLN1 protein, indicating the anti-fibrotic efficacy. For reference, in intestinal fibrosis, myofibroblasts transition into myofibroblasts and produce excessive fibrous tissue.

In particular, among the compositions of the present disclosure, the sample with rhHAPLN1 protein content of 10 ng/ml showed the most excellent anti-fibrotic efficacy, even though the content was 1/10,000th of that of pirfenidone at 100 µg/mL (100,000 ng/mL), rhHAPLN1 inhibited the extent of TGFβ1-induced αSMA by 71.6% ((3.82-1.8)/(3.82-1)*100), while pirfenidone showed a 10.6% inhibition ((3.82-3.52)/(3.82-1)*100), when comparing the efficacy of the drugs, the anti-fibrotic efficacy of rhHAPLN1 was approximately 7 folds higher, and although the content was 1/20,000th of that of pirfenidone at 200 µg/mL (200,000 ng/ml), the anti-fibrotic efficacy was much higher than twice that of pirfenidone at 200 µg/mL (200,000 ng/ml).

Since 200 µg/mL of pirfenidone shows a higher expression level of αSMA than 100 µg/mL, the anti-fibrotic efficacy is lower at high concentrations, and it may be assumed that 100 µg/mL is the optimal anti-fibrotic concentration of pirfenidone. If so, it may be seen that the composition of the present disclosure has superior anti-fibrotic efficacy compared to pirfenidone, a commercially available anti-fibrotic agent.

### [Example 4]

### Evaluation of anti-fibrotic efficacy of composition of present disclosure on human microvascular endothelial cell

Human cardiac microvascular endothelial cell (HCMEC) was used as a sample to verify the anti-fibrotic action of the composition of the present disclosure on cardiac fibrosis.

### 1. Experimental method

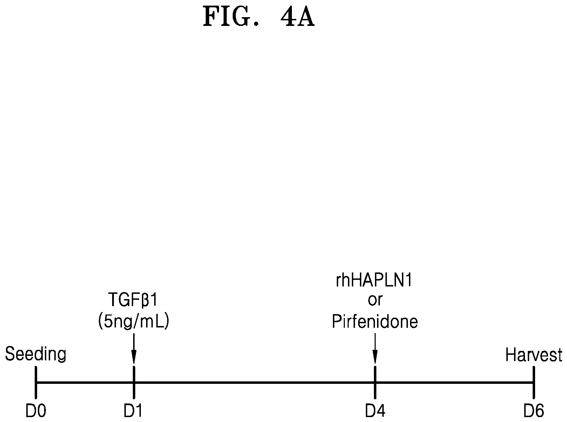

(1) Primary human cardiac microvascular endothelial cell (HCMEC; ScienCell, #6000) were seeded (5.7 × 10⁴ cells/well) in a 6-well plate according to the experimental scheme of FIG. 4A and incubated in Endothelial cell medium (ScienCell) for 24 hours in an incubator at 37 °C 5 % CO₂.
(2) The plates were then treated with TGFβ1 (5 ng/ml) to induce fibrosis and incubated for 3 days.
(3) Afterwards, each well of the plate was then treated with the composition of the present disclosure at rhHAPLN1 protein content of 0, 3, 10, 30, and 100 ng/ml and pirfenidone 100 µg/mL and 200 µg/mL, respectively, and incubated for 3 days.
(4) Each sample was then treated with cell lysis buffer and harvested.
(5) Western blotting was performed on the samples. Here, anti-αSMA Ab (Abcam, ab7817), anti-GAPDH Ab (Santa Cruz, sc-32233) were used as primary antibodies and HRP-conjugated anti-mouse IgG Ab (CST, #7076) as secondary antibodies to confirm the protein expression levels of αSMA and GAPDH.

### 2. Result

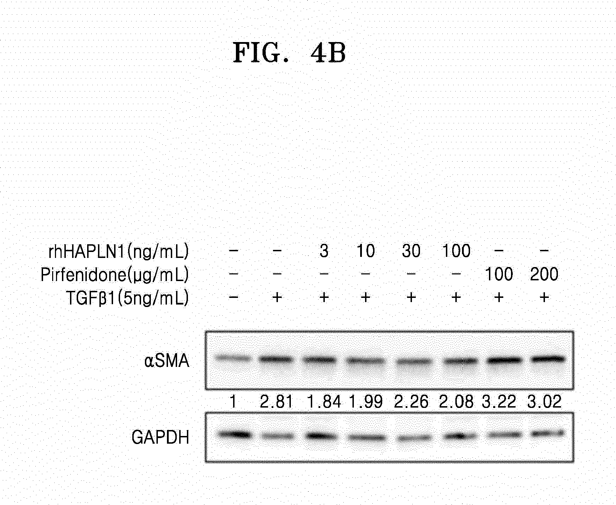

In FIG. 4B, it may be seen from the western blot band photographs that the composition of the present disclosure exhibits much better anti-fibrotic action compared to pirfenidone in all rhHAPLN1 protein content ranges, as indicated by the darkness and thickness of the αSMA protein band, in terms of the extent of fibrosis of human cardiac microvascular endothelial cells.

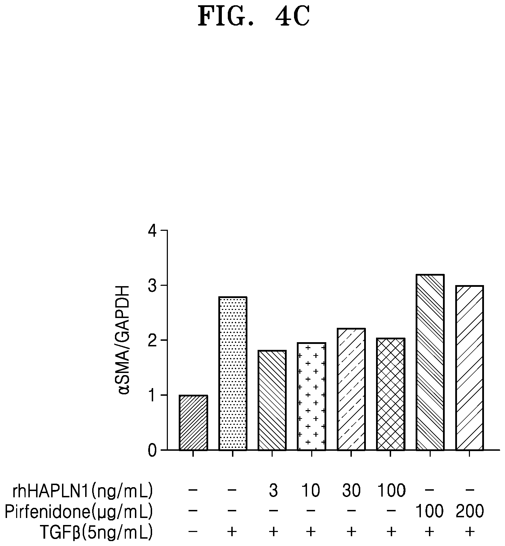

Furthermore, FIG. 4C shows that after inducing fibrosis with TGFβ1 (5 ng/ml), the expression level of αSMA as a myofibroblast marker was significantly lower in all rhHAPLN1 protein content ranges when treated with the composition of the present disclosure compared to when treated with the composition that does not include rhHAPLN1 protein, indicating the anti-fibrotic efficacy. For reference, in cardiac fibrosis, endothelial cells transition into myofibroblasts and produce excessive fibrous tissue.

In particular, among the compositions of the present disclosure, the sample with rhHAPLN1 protein content of 3 ng/ml showed the most excellent anti-fibrotic efficacy, which showed an anti-fibrotic efficacy that was not found in pirfenidone, even though the content was 3/100,000th of that of pirfenidone at 100 µg/mL (100,000 ng/mL), and although the content was 3/200,000th of that of pirfenidone at 200 µg/mL (200,000 ng/ml), the sample showed anti-fibrotic efficacy that were not found in pirfenidone.

No matter what concentration of pirfenidone is treated, the expression level of αSMA was higher than when treated with a composition that does not include rhHAPLN1 protein, so it may be seen that the anti-fibrotic efficacy is lower in cardiac microvascular endothelial cells. This suggests that the composition of the present disclosure may be an almost unique anti-fibrotic agent, at least for cardiac microvascular endothelial cells.

### [Example 5]

### Evaluation of anti-fibrotic efficacy of composition of present disclosure on normal human lung fibroblast

Normal human lung fibroblast (NHLF) was used as a sample to verify the anti-fibrotic action of the composition of the present disclosure on pulmonary fibrosis.

### 1. Experimental method

(1) normal human lung fibroblasts (NHLF) cultured for 9 passages were seeded (1.0 × 10⁵ cells/well) in a 6-well plate and incubated in FBM-2 (Fibroblast Growth Medium-2, CC-3131, Lonza) medium for 24 hours in an incubator at 37 °C 5 % CO₂.
(2) After replacing with serum-free medium, the medium was incubated for 24 hours.
(3) In this regard, under conditions in which fibrosis was induced by treatment with TGFβ1 (10 ng/ml), each well of the plate was treated with the composition of the present disclosure with rhHAPLN1 protein content of 0, 3.1, 12.5, 25 and 100 ng/ml and 100 µg/mL and 200 µg/mL of pirfenidone (Selleckchem), respectively, and incubated for 24 hours.
(4) Afterwards, each sample was washed twice with phosphate buffered saline (PBS, pH 7.2), and then the expression level of αSMA, a marker for myofibroblasts, was confirmed by western blot.

### 2. Result

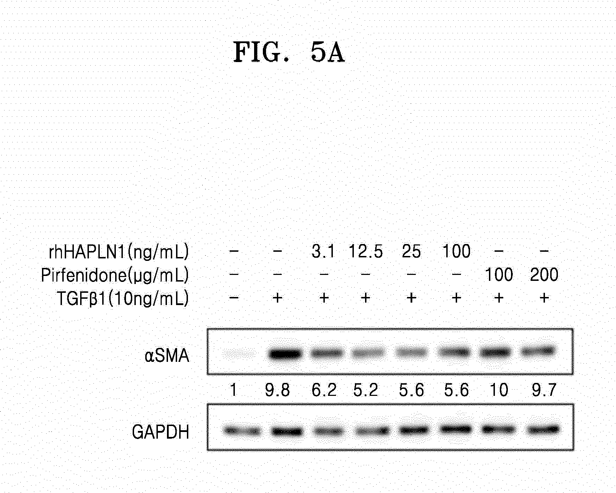

In FIG. 5A, it may be seen from the western blot band photographs that the composition of the present disclosure exhibits much better anti-fibrotic action compared to pirfenidone in all rhHAPLN1 protein content ranges, as indicated by the darkness and thickness of the αSMA protein band, in terms of the extent of fibrosis of normal human lung fibroblasts.

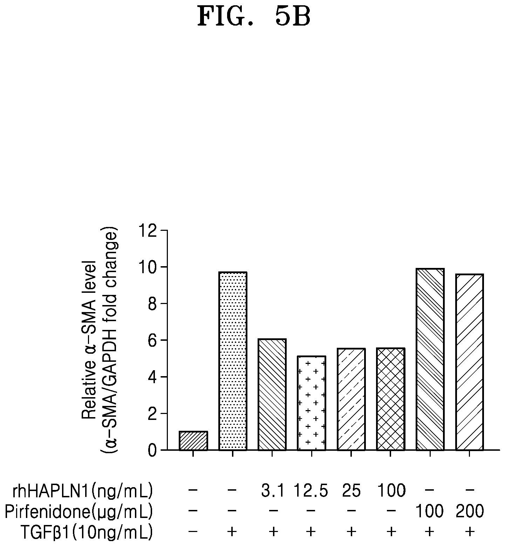

Furthermore, FIG. 5B shows that after inducing fibrosis with TGFβ1 (10 ng/ml), the expression level of αSMA as a myofibroblast marker was significantly lower in all rhHAPLN1 protein content ranges when treated with the composition of the present disclosure compared to when treated with the composition that does not include rhHAPLN1 protein, indicating the anti-fibrotic efficacy.

In particular, among the compositions of the present disclosure, the sample with rhHAPLN1 protein content of 12.5 ng/ml showed the most excellent anti-fibrotic efficacy, which was about twice as higher in anti-fibrotic efficacy even though the content was 1/10,000th of that of pirfenidone at 100 µg/mL (100,000 ng/ml), and about twice as higher than that of pirfenidone at 200 µg/mL (200,000 ng/ml) as well.

No matter what concentration pirfenidone is treated with, there is little difference in the expression level of αSMA compared to when treated with a composition that does not include rhHAPLN1 protein, indicating that pirfenidone has no anti-fibrotic effect, at least in lung fibroblasts. This suggests that the composition of the present disclosure may be an almost unique anti-fibrotic agent, for lung fibroblasts.

### [Example 6]

### Evaluation of fibrosis prevention efficacy of composition of present disclosure on human kidney renal tubule cell

An epithelial cell line of human kidney renal tubule cell (Human Kidney 2, HK-2) was used as a sample to verify the fibrosis prevention action of the composition of the present disclosure on kidney fibrosis.

### 1. Experimental method

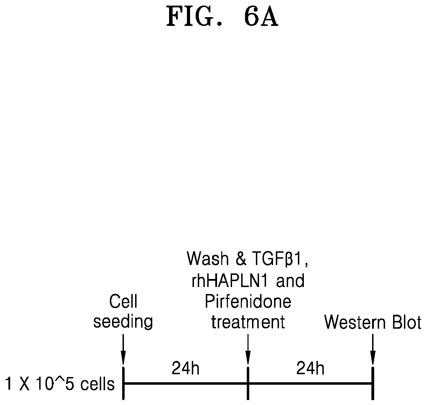

(1) Human kidney renal tubule cells (HK-2) were seeded (1.0 × 10⁵ cells/well) in a 6-well plate according to the experimental scheme of FIG. 6A and incubated in RPMI 1640 Medium (11875-093, Gibco) including 10 % FBS for 24 hours in an incubator at 37 °C 5 % CO₂.
(2) The plates were then washed several times with PBS and each well of the plate was simultaneously treated with TGFβ1 (5 ng/ml) and the composition of the present disclosure (rhHAPLN1 protein content of 0, 5, 10, 20, and 50 ng/ml) and pirfenidone 0.2 mg/mL (200 µg/mL) and incubated for 24 hours.
(3) Each sample was then washed several times with ice-cold PBS, treated with radioimmunoprecipitation assay (RIPA) lysis and extraction buffer (Thermo Scientific) including protease and phosphatase inhibitors, scraped with a scraper, and transferred to an E-tube.
(4) By centrifugation (12000 rpm, 20 minutes, 4 °C), only the supernatant was obtained, and bicinchoninic acid (BCA) assay was performed to obtain quantitative protein values.
(5) After quantification using the obtained quantitative value, cooking was performed at 100 °C for 3 minutes and then stored in a -20 °C refrigerator.
(6) Using a 4 % to 15 % precast acrylamide PAGE gel, proteins were loaded at 10 µg per lane and run (80V - 20 minutes, 120V - 1 hour 30 minutes).
(7) Changed to 100V - 1 hour and 30 minutes and blocked with a smart blocker for about 5 minutes.
(8) The primary antibody was diluted 1:1000 in 1 % BSA-TBST, dispensed, and reacted overnight at 4 °C.
(9) The next day, the blot was washed three times for 10 minutes each with TBST.
(10) The secondary antibody was diluted 1:2000 in 1 % BSA-TBST (Bovine Serum Albumin-Tris-Buffered Saline & Polysorbate 20), dispensed, and shaken for about 1 hour at room temperature.
(11) After washing three times with TBST for 10 minutes each, the samples were detected by Chemidoc.

### 2. Result

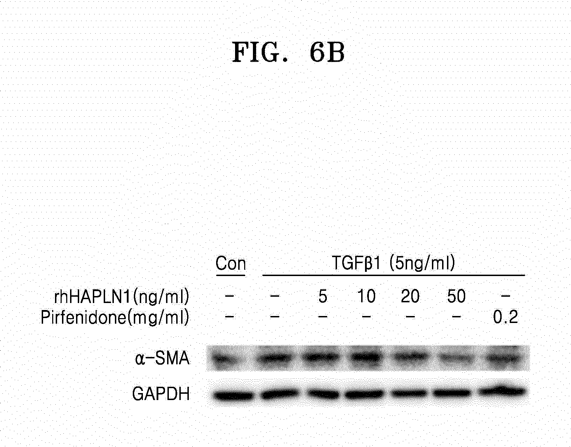

In FIG. 6B, it may be seen from the western blot band photographs that the composition of the present disclosure exhibits superior fibrosis prevention action compared to pirfenidone in a certain rhHAPLN1 protein content range (rhHAPLN1 protein content of 50 ng/ml), as indicated by the darkness and thickness of the αSMA protein band, in terms of the extent of fibrosis of human kidney renal tubule cells.

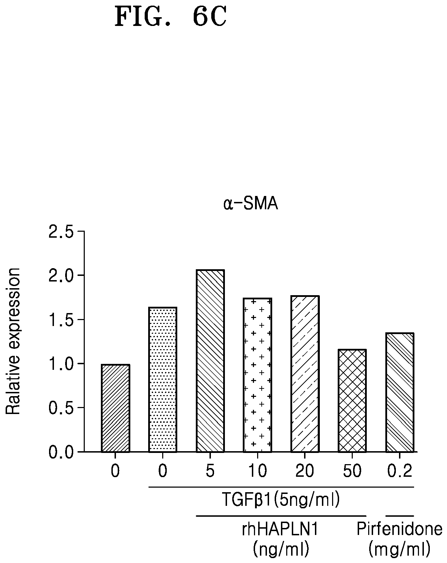

Furthermore, in FIG. 6C, after inducing fibrosis with TGF-β (5 ng/ml), treatment with the composition of the present disclosure at a rhHAPLN1 protein content of 50 ng/ml showed superior fibrosis prevention efficacy compared to treatment with the composition that does not include rhHAPLN1 protein, although the content was 1/4,000th of that of pirfenidone at 200 µg/mL (200,000 ng/ml), the fibrosis prevention efficacy was shown to be significantly higher.

### [Example 7]

### Evaluation of anti-fibrotic efficacy of composition of present disclosure on human kidney renal tubule cell

An epithelial cell line of human kidney renal tubule cell (Human Kidney 2, HK-2) was used as a sample to verify the anti-fibrotic action of the composition of the present disclosure on kidney fibrosis.

### 1. Experimental method

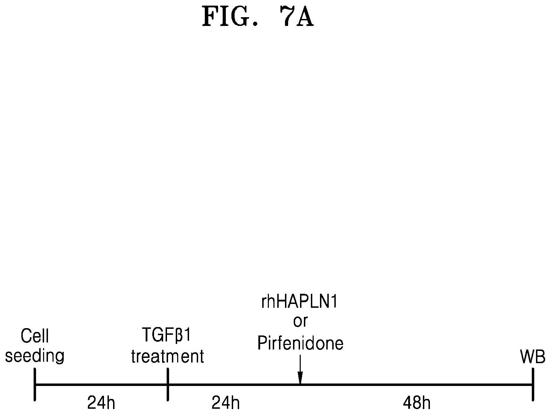

(1) Human kidney renal tubule cells (HK-2) were seeded (2.8 × 10⁵ cells/well) in a 60-well plate according to the experimental scheme of FIG. 7A and incubated in RPMI 1640 Medium (11875-093, Gibco) including 10 % FBS for 24 hours in an incubator at 37 °C 5 % CO₂.
(2) Afterwards, the plates were washed several times with PBS, and each well of the plate was treated with TGFβ1 (5 ng/ml) and incubated for 24 hours.
(3) Subsequently, the plates were then treated with the composition of the present disclosure (rhHAPLN1 protein content of 0, 5, 10, 20, and 50 ng/ml) and pirfenidone 0.2 mg/mL (200 µg/mL), respectively, and incubated for 48 hours.
(4) Each sample was then washed several times with ice-cold PBS, treated with radioimmunoprecipitation assay (RIPA) lysis and extraction buffer (Thermo Scientific) including protease and phosphatase inhibitors, scraped with a scraper, and transferred to an E-tube.
(5) By centrifugation (12000 rpm, 20 minutes, 4 °C), only the supernatant was obtained, and BCA assay was performed to obtain quantitative protein values.
(6) After quantification using the obtained quantitative value, cooking was performed at 100 °C for 3 minutes and then stored in a -20 °C refrigerator.
(7) Using a 8 % precast acrylamide PAGE gel, proteins were loaded at 10 µg per lane and run (80V - 20 minutes, 120V - 1 hour 30 minutes).
(8) Changed to 100V - 1 hour and 30 minutes and blocked with a smart blocker for about 5 minutes.
(9) The primary antibody was diluted 1:1000 in 1 % BSA-TBST, dispensed, and reacted overnight at 4 °C.
(10) The next day, the blot was washed three times for 10 minutes each with TBST.
(10) The secondary antibody was diluted 1:2000 in 1 % BSA- TBST, dispensed, and shaken for about 1 hour at room temperature.
(11) After washing three times with TBST for 10 minutes each, the samples were detected by Chemidoc.

### 2. Result

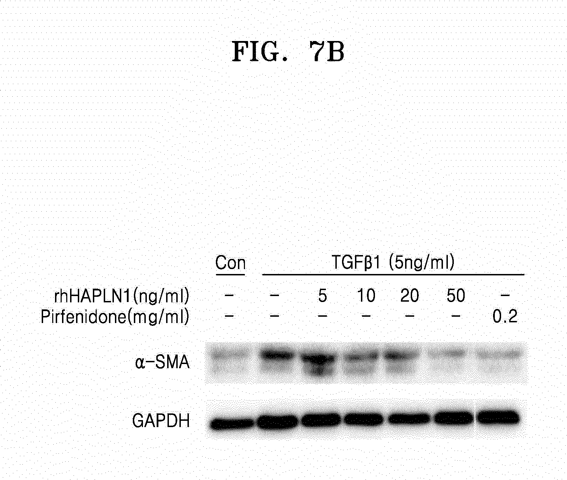

In FIG. 7B, it may be seen from the western blot band photographs that the composition of the present disclosure exhibits superior anti-fibrotic action in a certain rhHAPLN1 protein content range (rhHAPLN1 protein content of 10 to 50 ng/ml), as indicated by the darkness and thickness of the αSMA protein band, in terms of the extent of fibrosis of human kidney renal tubule cells.

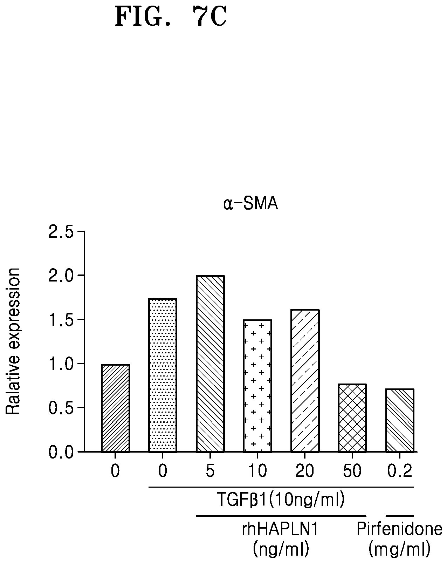

Furthermore, FIG. 7C shows that after inducing fibrosis with TGF-β (10 ng/ml), the compositions of the present disclosure exhibited excellent anti-fibrotic efficacy when treated with rhHAPLN1 protein content of 10 to 50 ng/ml, especially when treated with rhHAPLN1 protein content of 50 ng/ml, compared to when treated with a composition that does not include rhHAPLN1 protein. It is quite noteworthy that treatment with 50 ng/ml of the composition of the present disclosure exhibited anti-fibrotic efficacy almost equivalent to that of pirfenidone, even though the composition was a small amount of 0.2 mg/ml, in other words, 1/4th of the amount compared to 200 ng/ml of pirfenidone.

### [Example 8]

### Observation of cell morphological changes following anti-fibrotic efficacy of composition of present disclosure on human kidney renal tubule cell.

An epithelial cell line of human kidney renal tubule cell (Human Kidney 2, HK-2) was used as a sample to verify the anti-fibrotic action of the composition of the present disclosure on kidney fibrosis by observing the morphological changes of the cell.

### 1. Experimental method

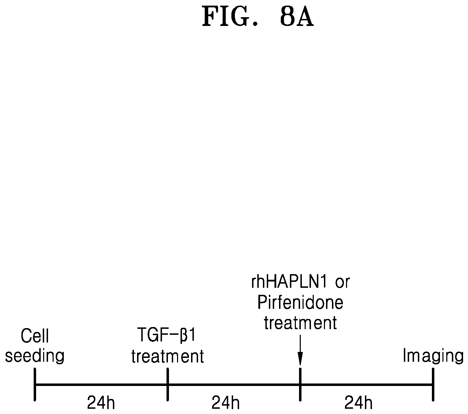

(1) Human kidney renal tubule cells (HK-2) were seeded (3.0 × 10⁵ cells/well) in a 6-well plate according to the experimental scheme of FIG. 8A and incubated in RPMI 1640 Medium (11875-093, Gibco) including 10 % FBS for 24 hours in an incubator at 37 °C 5 % CO₂.
(2) Afterwards, the plates were washed several times with PBS, and each well of the plate was treated with TGFβ1 (10 ng/ml) and incubated for 24 hours.
(3) Subsequently, the plates were then treated with the composition of the present disclosure (rhHAPLN1 protein content of 0, 5, 10, 20, 50 and 100 ng/ml) and pirfenidone 0.2 mg/mL (200 µg/mL), respectively, and incubated for 24 hours.
(4) The wells containing each sample were then washed twice with 1X PBS, treated with 4 % paraformaldehyde and fixed for 1 hour.
(5) After washing twice with 1X PBS, the shape or morphology of the cells was observed under a microscope.

### 2. Result

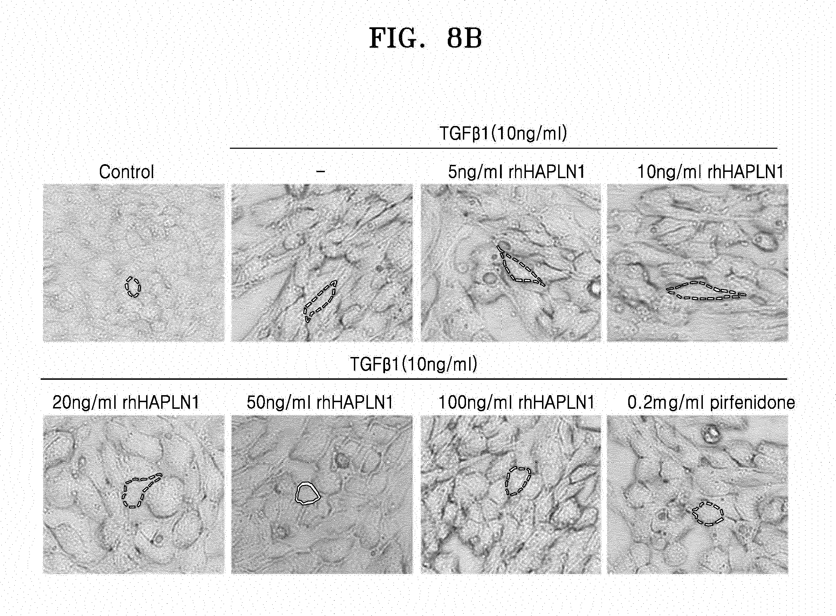

In FIG. 8B, the morphology of the cells varies depending on the treated substance. It was confirmed that in the control, which were normal cells without any treatment, the cells appeared round in shape, but upon treatment with 10ng/ml TGFβ1, the round cell shape changed into a sharp fibrotic form.

However, as the composition of the present disclosure with various rhHAPLN1 protein contents was added, the shape of the cells changed to round, similar to the case of pirfenidone.

Accordingly, it may be seen that the composition of the present disclosure clearly exhibited excellent anti-fibrotic efficacy against cells.

### [Example 9]

### Evaluation of anti-fibrotic efficacy of composition of present disclosure on renal proximal tubule epithelial cell 1

The primary epithelial cell of renal proximal tubule epithelial cell (RPTEC) were used as samples to verify the anti-fibrotic action of the composition of the present disclosure against age-induced kidney fibrosis.

### 1. Experimental method

(1) Renal proximal tubule epithelial cell (RPTEC) of the 3rd passage culture and 10th passage culture were seeded (5.0 × 10⁵ cells) in 60Φ culture dishes and cultured in complete renal epithelial cell medium (ATCC PCS-400-030+ATCC PCS-400-040) in an incubator at 37 °C 5 % CO₂ for 24 hours.
(2) Subsequently, the cells were then treated with the composition of the present disclosure (rhHAPLN1 protein content of 0, 10, and 100 ng/ml, respectively) and cultured for an additional 72 hours.
(4) After washing three times with ice-cold PBS, each well was treated with 300 µl of RIPA lysis and extraction buffer including protease inhibitors and phosphatase inhibitors, scraped with a scraper to harvest cells, and transferred to an E-tube.
(5) By centrifugation (12000 rpm, 20 minutes, 4 °C), only the supernatant was obtained, and BCA assay was performed to obtain quantitative protein values.
(6) Using a 4 % to 15 % precast acrylamide PAGE gel, proteins were loaded at 20 µg per lane and run (80V - 20 minutes, 120V - 1 hour 30 minutes).
(7) Western blot was performed on the samples. Here, anti-αSMA Ab (Abeam, ab7817), anti-GAPDH Ab (Santa Cruz, sc-32233) were used as primary antibodies and HRP-conjugated anti-mouse IgG Ab (CST, #7076) as secondary antibodies.

### 2. Result

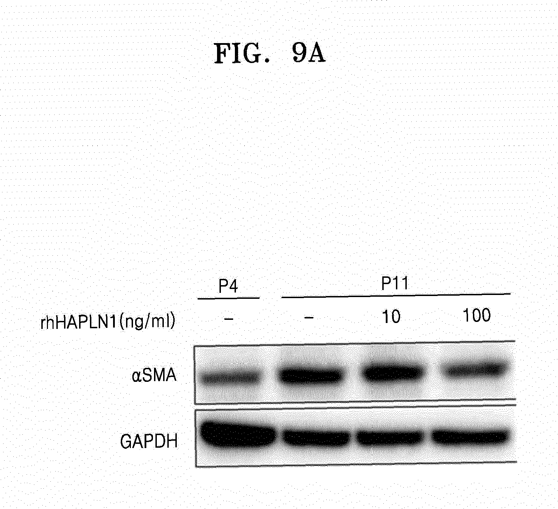

As shown in FIG. 9A, as cell aging progresses, in other words, as the number of passage culture increases from 4 to 11, the αSMA protein expression level increases, which may be seen from the intensity and thickness of the protein band in the western blot band photograph.

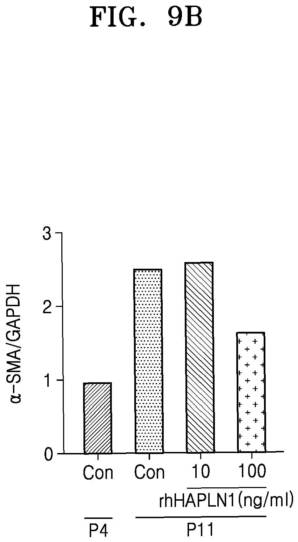

Furthermore, in FIG. 9B, it is shown that in aged cells (P11), the expression amount of αSMA protein decreased by about 1/3rd when treated with the composition of the present disclosure at a rhHAPLN1 protein content of 100 ng/ml compared to when treated with the composition that does not include rhHAPLN1 protein. In other words, the composition showed excellent anti-fibrotic efficacy.

### [Example 10]

### Evaluation of anti-fibrotic efficacy of composition of present disclosure on renal proximal tubule epithelial cell 2

The primary epithelial cell of renal proximal tubule epithelial cell (RPTEC) were used as samples to verify the anti-fibrotic action of the composition of the present disclosure against age-induced kidney fibrosis.

### 1. Experimental method

(1) Renal proximal tubule epithelial cell (RPTEC) of the 3rd passage culture and 10th passage culture were seeded (1.5 × 10⁵ cells) in 12-well plates and cultured in complete renal epithelial cell medium (ATCC PCS-400-030+ATCC PCS-400-040) in an incubator at 37 °C 5 % CO₂ for 24 hours.
(2) Subsequently, the plates were then treated with the composition of the present disclosure (rhHAPLN1 protein content of 0, 10, 20, 50 and 100 ng/ml) and pirfenidone 0.2 mg/mL (200 µg/mL), respectively, and cultured for 72 hours.
(3) Afterwards, each well was washed twice with PBS, then treated with 4 % paraformaldehyde and fixed for 1 hour.
(4) After washing four times with 1X PBS, the wells were treated with 1x PBST (1x PBS + 0.1 % Triton X-100) containing 1 % BSA and blocked for 2 hours.
(5) Primary antibody against αSMA (Abeam ab7817) was diluted 1:200 in 1XPBSTdp and treated and incubated overnight at 4 °C.
(6) The next day, the blot was washed four times with 1X PBST.
(7) After treatment with secondary antibody (anti-mouse IgG Alexa Fluor^{™} 488 1:500 (Invitrogen, A28175)) and DAPI 1:2000 (Invitrogen, D1306), the cells were incubated at room temperature for 1 hour.
(8) Afterwards, 50ul of Vectashield (Vectorlabs, H-1000) was added, covered with a coverslip, and photographed with a fluorescence microscope.

### 2. Result

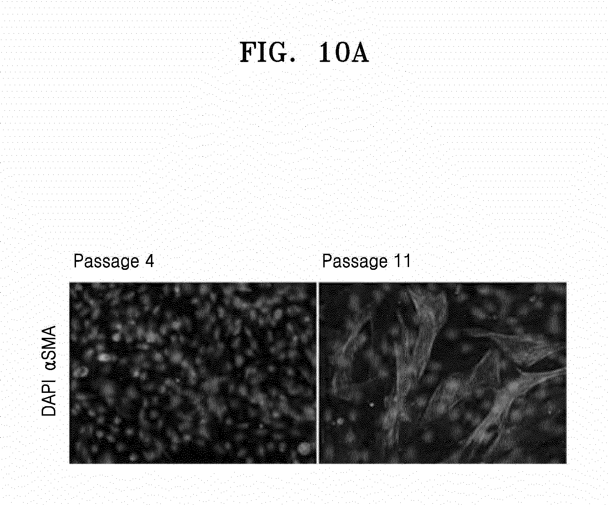

As shown in FIG. 10A, as cell aging progresses, that is, as the number of passage culture increases from 4 to 11, the expression level of αSMA protein increases, in other words, the advancement of fibrosis may be confirmed from the increased bright fluorescence area in the right photograph of the 11th passage culture.

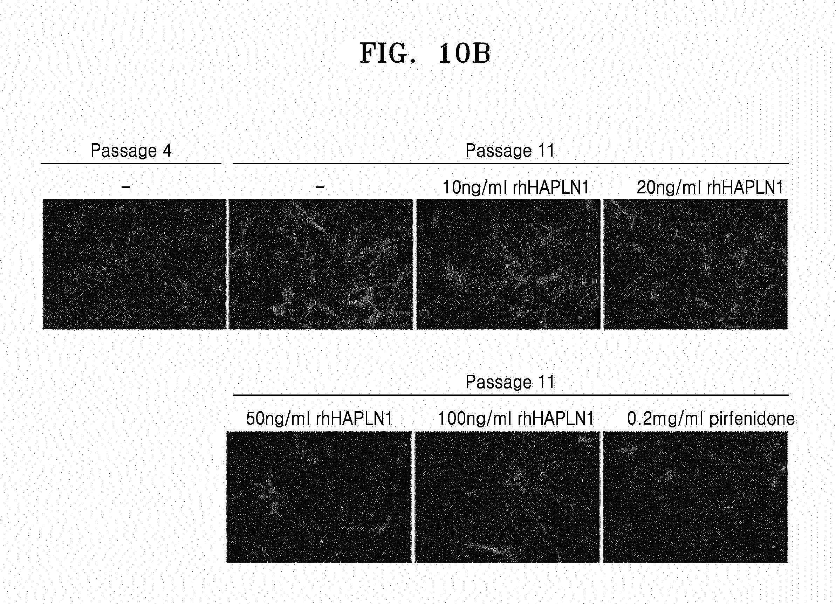

In addition, in FIG. 10B, it was confirmed that when treated with a composition of the present disclosure in the case of aged cells (11th passage culture) as compared to when treated with a composition that does not include rhHAPLN1 protein, as the content of rhHAPLN1 protein increased, especially for rhHAPLN1 protein content of 50 ng/ml and 100 ng/ml, fewer stained fluorescent areas appeared, as in the case of treatment with pirfenidone. However, considering that the content of pirfenidone is 0.2 mg/mL (200,000 ng/mL), and therefore the rhHAPLN1 protein content in the composition of the present disclosure is only 4,000th to 2,000th of that of pirfenidone, it may be seen that the composition of the present disclosure exhibited excellent anti-fibrotic efficacy compared to usual anti-fibrotic agents.

### [Example 11]

### Evaluation of anti-fibrotic efficacy of composition of present disclosure using various anti-fibrotic markers

The anti-fibrotic action effect of the composition of the present disclosure on pulmonary fibrosis and kidney fibrosis was verified by examining the expression level of various fibrosis markers using the BioMAP Fibrosis Panel (provided by Eurofins discovery).

BioMAP Fibrosis panel consists of three models, SAEMyoF system (co-culturing small airway epithelial cells and adult fibroblasts), REMyoF system (co-culture of renal proximal tubule epithelial cells and adult fibroblasts), and MyoF system (differentiated lung myofibroblasts). Here, the SAEMyoF system represents interstitial lung diseases (IDL), including pulmonary fibrotic diseases such as idiopathic pulmonary fibrosis (IPF), and the REMyoF system represents kidney fibrosis disease, which is associated with end-stage renal failure. However, experiments were conducted to confirm the effect of the MyoF system only on myofibroblasts.

### 1. Experimental method

(1) Each primary cell was used by pooling cells from multiple donors (n=3 to 6), and cells were first cultured in 96-well plates to confluence before adding a peripheral blood mononuclear cell (PBMC).
(2) Subsequently, the cells were then each treated with the composition of the present disclosure (rhHAPLN1 protein content of 0, 3.7, 11, and 33 ng/ml) at different concentrations and cultured for 1 hour.
(3) The plates were then treated with TNFα and TGFβ1 and incubated for 48 hours.
(4) Direct enzyme-linked immunosorbent assay (ELISA) was performed on the expression levels of various biomarkers.

### 2. Result

Each quantitative value was compared as a relative fold change compared to the vehicle control. Statistical processing was performed using unpaired t-test, significant differences vs vehicle control, *p<0.05, **p<0.01, and ***p<0.001.

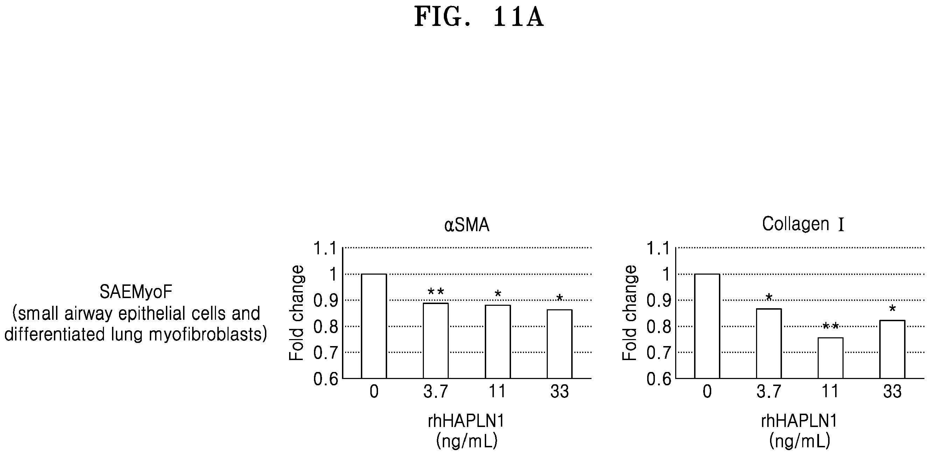

According to the experimental results, a significant reducing effect of the composition of the present disclosure (rhHAPLN1) on αSMA and collagen I was confirmed in the SAEMyoF system, a pulmonary fibrosis disease model (FIG. 11A). That is, in the case of αSMA, it was confirmed that the expression level of αSMA was consistently reduced to 0.9 fold or less when treated with the composition of the present disclosure compared to when treated with a composition that does not include rhHAPLN1 protein, regardless of the content of rhHAPLN1 protein in the composition. In addition, in the case of collagen I, it was confirmed that the expression level of collagen I was consistently significantly reduced when treated with the composition of the present disclosure compared to when treated with a composition that does not include rhHAPLN1 protein, regardless of the content of rhHAPLN1 protein in the composition. In particular, when the rhHAPLN1 protein content in the composition was 11 ng/ml, the expression level of collagen I was reduced to 0.8 fold or less.

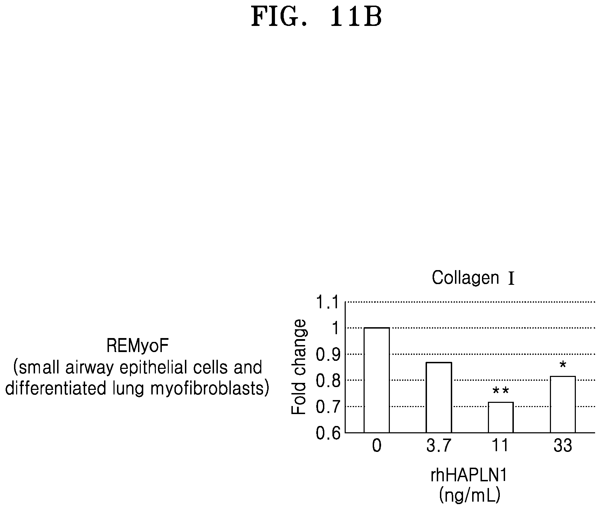

Furthermore, in the REMyoF system, a kidney fibrosis disease model, a significant reduction effect of the composition of the present disclosure (rhHAPLN1) on collagen I was confirmed (FIG. 11B). That is, it was confirmed that the expression level of collagen I was reduced to 0.9 fold or less when treated with the composition of the present disclosure compared to when treated with a composition that does not include rhHAPLN1 protein, regardless of the content of rhHAPLN1 protein in the composition. In particular, when the rhHAPLN1 protein content in the composition was 11 ng/ml, the expression level of collagen I was reduced by almost 0.7 fold, and it may be assumed that this concentration was the optimal concentration for demonstrating anti-fibrotic efficacy in the kidney.

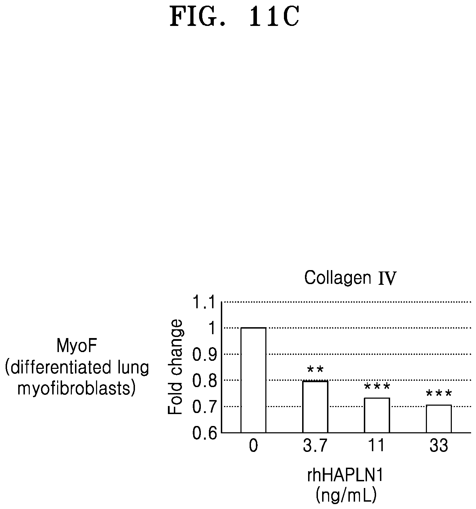

Meanwhile, in the MyoF system related to myofibroblasts, a significant reducing effect of the composition of the present disclosure (rhHAPLN1) on collagen IV was confirmed (FIG. 11c). That is, it was confirmed that when treated with the composition of the present disclosure compared to the case treated with a composition that does not include rhHAPLN1 protein, the expression level of collagen I was reduced by 0.8 fold or less in proportion to the content of rhHAPLN1 protein in the composition. Therefore, when the rhHAPLN1 protein content in the composition was 33ng/ml, the expression level of collagen IV was reduced by 0.7 fold.

### [Example 12]

### Evaluation of anti-fibrotic efficacy (prevention of fibrotic disease) of composition of present disclosure using animal model inducing pulmonary fibrosis

An experiment was conducted to verify the effect of preventing bleomycin-induced pulmonary fibrosis (BIPF) by repeatedly administering the composition of the present disclosure by inhalation to animals with pulmonary fibrosis.

### 1. Preparation and raising of experimental animal

The experimental animals used were 8-week-old female C57BL/6N (KBSI, Daejeon, Korea) mice, and were set as a normal control group (Normal, PBS) and two dose of administration groups (composition of the present disclosure including rhHAPLN1 protein). The water was changed every two days and the mice were allowed to eat ad libitum, and the mice were placed in one cage of four, and the temperature in the room was kept at 21 °C to 24 °C and the humidity at 40 % to 60 %, and the day and night cycles were 12 hours each.

### 2. Staining and microscopic observation of lung tissue

To establish a bleomycin-induced pulmonary fibrosis (PF) model, 8-week-old female C57BL/6N mice were used, and divided into 4 groups (Normal, Control, 0.0005 % (w/w) rhHAPLN1, 0. 0015 % (w/w) rhHAPLN1), with 4 mice per group, and the IPF disease induction method was referred to the paper by Liu et al. (Methods in Molecular Biology 2017, DOI: 10.1007/978-1-4939-7113-8_2) (see FIG. 12A). Administration of bleomycin (Bleomycin sulfate from Streptomyces verticillus. B8416; Sigma-Aldrich Co.) to induce idiopathic pulmonary fibrosis (IPF) was performed using a 1 ml-syringe and an oral dosing needle (Oral Zonde Needle 0. 9x50mm; 20Guage) was used to anesthetize the patient and then carefully instillated orally intratracheally in a single 25 µl dose at a concentration of 2 U/kg, followed by nebulization with fresh 10 ml phosphate-buffered saline (1X PBS, pH 7.4; Gibco Co.) added dropwise to a nebulizer to generate nebulization and be freely inhaled.

Starting 1 day after bleomycin administration, the control was a mixture of 50 µl of a stock solution consisting of 20 mM Tris-HCl, 0.5 M NaCl, pH 8.0, 50 % glycerol and 10 ml PBS (1X, pH 7.4, Gibco Co.) solution, while the two groups for the compositions of the present disclosure were treated with 50 µl of rhHAPLN1 stock solution at a rhHAPLN1 weight ratio concentration of 0. 0005 % (w/w), in other words, 5,000 ng/mL, and 0.0015 % (w/w), in other words, 15,000 ng/mL, respectively, were mixed in 10 ml PBS (1X, pH 7.4, Gibco Co.) and administered to each group once a day for 1 hour by nebulization via the nebulizer described above. The weight of each experimental animal was measured every 2 days to confirm that there was no change in weight, and a mass dosing system and aerosol chamber (Data Science International Co.) were used to generate the mist.

The total experiment period was 12 days, and the actual administration of recombinant HAPLN1 protein at each concentration was administered once daily for 1 hour for 10 days, and then dissection was performed after anesthesia on day 11.

### 3. Result

### A. Photographic comparison of lung tissue

FIG. 12B is a photograph of lung tissue harvested, then the left large lobe was cut in half transversely, the upper portion was fixed in formalin, and hematoxylin and eosin (H&E) staining was performed. When looking at the photograph, it may be seen that when the composition of the present disclosure was administered compared to the control group, especially at a rhHAPLN1 weight ratio concentration of 0.0015% (w/w), the form was quite similar to that of the normal group.

### B. Preventive efficacy of composition of present disclosure expressed in numbers

Next, three tissue slides were prepared per mouse by cutting the middle portion of the left large lobe of the mouse, and three sections were photographed randomly on one slide, and then, for a total of nine sections, the stained darker part was distinguished from the unstained white part, and the area of the stained darker part was measured using Image J software, and each figure obtained was averaged to obtain an average value per mouse. The dark area indicate areas of advanced fibrosis.

Furthermore, the same method was repeated for the remaining three animals to obtain the average value, which was then re-averaged again to finally obtain the mean value and deviation of the area reflecting fibrosis. The *p*-value of significance between the normal group not treated with bleomycin and the control treated with bleomycin was 0.00038 (****p*< 0.001), and the *p*-value of significance between the group not treated with the composition of the present disclosure (rhHAPLN1) and the group treated with 0.0015 % (w/w) rhHAPLN1 was 0.00355 (***p*< 0.01), confirming statistical significance.

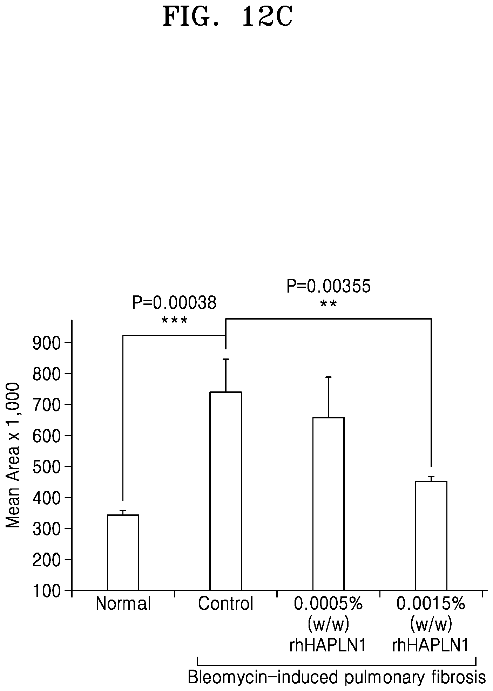

According to FIG. 12C, which shows these average values in a graph, it can be seen that when the composition of the present disclosure is administered compared to the control group, especially when the rhHAPLN1 weight ratio concentration was 0.0015%(w/w), the average value of the area of the dark area was reduced to be quite close to the normal group. In other words, it was confirmed that the composition of the present disclosure exhibits a preventive effect against pulmonary fibrosis in vivo.

### C. Preventive efficacy of composition of present disclosure according to Ashcroft score

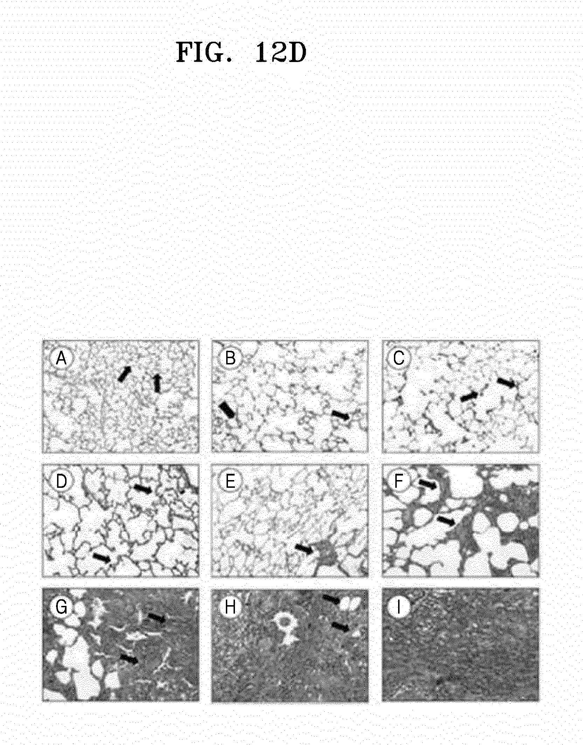

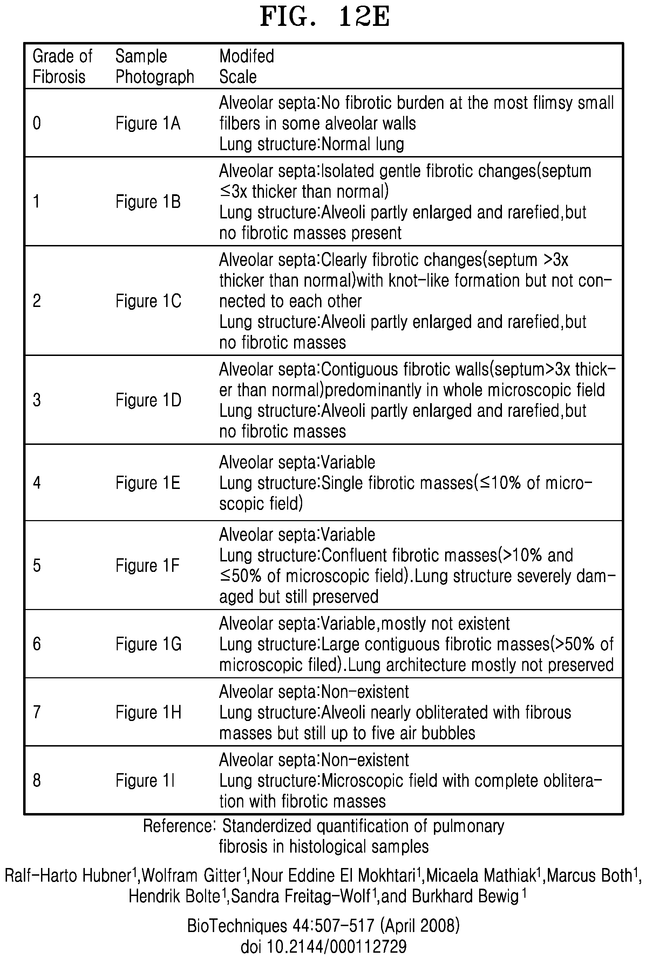

FIG. 12D and FIG. 12E are photographs and descriptions illustrating official guidelines for simply visually measuring the severity of pulmonary fibrosis (Ashcroft et al 1988, J Clin Pathol 41:467-470).

The grade of fibrosis can be divided into Ashcroft scores as follows.

**Score 0:** No obvious fibrotic morphology or burden is seen, although mostly thin, small fibers are observed in some alveolar walls (normal lung).

**Score 1:** Isolated soft fibrotic change (septal thickness less than or equal to 3 times normal), partially enlarged and sparse alveoli, but no fibrotic masses are present.

**Score 2*:*** Clear fibrotic change (septal thickness is greater than or equal to 3 times normal), alveoli are partially enlarged and sparse, but no fibrotic masses are observed.

**Score 3:** The entire microscopic area shows mainly continuous fibrotic septa (septal thickness is greater than 3 times normal), with partially enlarged and sparse alveoli, but no fibrotic masses are present.

**Score 4:** Structural change, single fibrotic masses are less than or equal to 10 % of the microscopic area.

**Score 5:** Structural change, combined fibrotic masses (greater than or equal to 10 % of the microscopic area and less than or equal to 50 %), lung structure is severely damaged but still preserves its shape.

**Score 6:** Structural change, most often absent. Continuous fibrotic mass (greater than 50 % of the microscopic area), mostly lung structure not preserved.

**Score 7:** Alveolar structure is absent. The alveoli have almost completely disappeared into a fibrous mass, but up to five air bubbles are still present.

**Score 8:** There is no alveolar structure. In the microscopic field, the fibrous mass was completely removed.

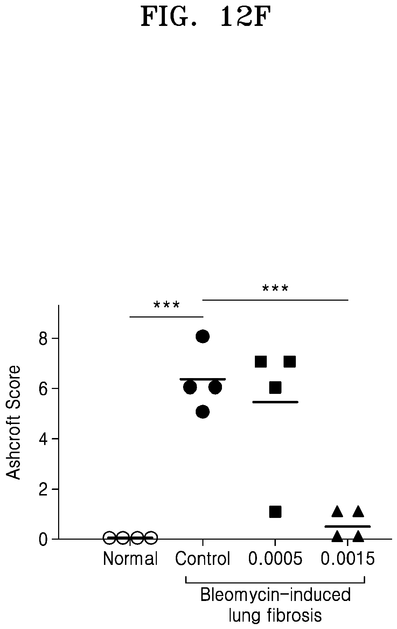

Based on the above evaluation criteria, three observers each scored the same data individually under the condition that they were not affected by the evaluation of others, and then summarized their results to confirm a final average score, and the results of each evaluator were very similar, and the results of two representative observers are shown in FIG. 12F.

In conclusion, the normal group had an Ashcroft score = 0, the control had an Ashcroft score = 6.25, the composition 1 (rhHAPLN1 0.0005 %) administration group of the present disclosure had an Ashcroft score = 5.25, and the composition 2 (rhHAPLN1 0.0015 %) administration group of the present disclosure had an Ashcroft score = 0.5, with statistical significance between the normal group and the control, and the control and the composition 2 administration group of the present disclosure.

### [Example 13]

### Evaluation of anti-fibrotic efficacy of composition of present disclosure using animal model of pulmonary fibrosis.

An experiment was conducted to verify the effect of ameliorating bleomycin-induced pulmonary fibrosis (BIPF) already developed by repeatedly administering the composition of the present disclosure by inhalation to animals with pulmonary fibrosis. Here, experimental results are presented that are basically similar to Example 12, but bleomycin-induced pulmonary fibrosis (BIPF) was sufficiently generated 7 days after bleomycin treatment, and that the composition of the present disclosure has the ability to treat fibrotic diseases.

### 1. Preparation and raising of experimental animal

Preparation and raising are the same as in Example 12 above.

### 2. Staining and microscopic observation of lung tissue

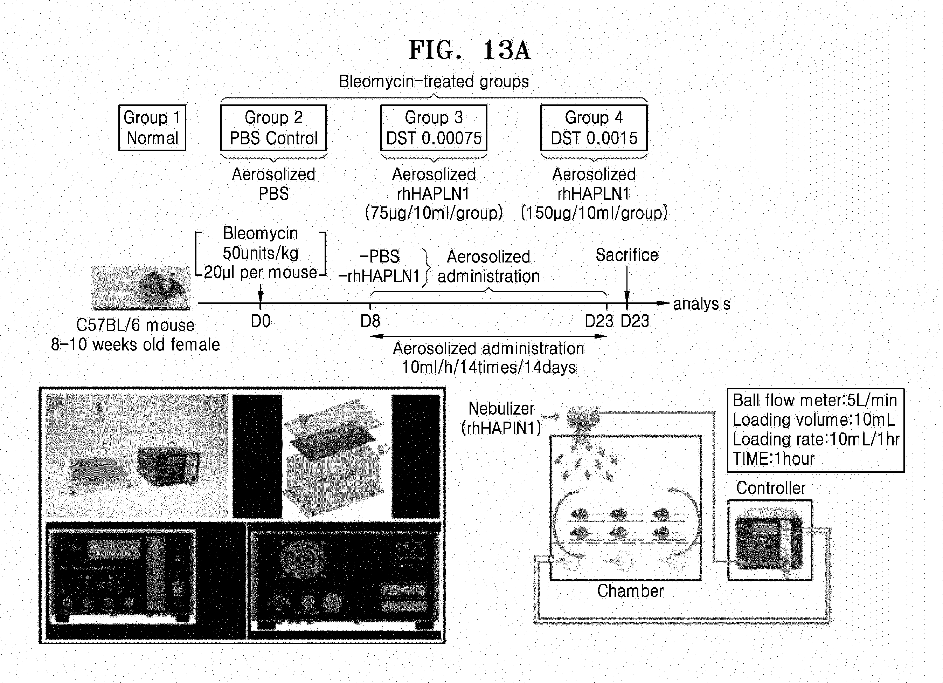

To establish a bleomycin-induced pulmonary fibrosis (PF) model, 8-week to 10-week-old female C57BL/6N mice were used, and divided into 4 groups (Normal, PBS Control, 0.00075 % (w/w) rhHAPLN1, 0. 0015 % (w/w) rhHAPLN1), with 4 mice per group, and the IPF disease induction method was referred to the paper by Liu et al. (Methods in Molecular Biology 2017, DOI: 10.1007/978-1-4939-7113-8_2) (see FIG. 13A). Administration of bleomycin (Bleomycin sulfate from Streptomyces verticillus. B8416; Sigma-Aldrich Co.) to induce idiopathic pulmonary fibrosis (IPF) was performed using a 1 ml-syringe and an oral dosing needle (Oral Zonde Needle 0. 9x50mm; 20Guage) was used to anesthetize the patient and then carefully instillated orally intratracheally in a single 20 µl dose per mouse at a concentration of 50 U/kg, followed by nebulization with fresh 10 ml phosphate-buffered saline (1X PBS, pH 7.4; Gibco Co.) added dropwise to a nebulizer to generate nebulization and be freely inhaled.

Starting 8 days after bleomycin administration, the control was a mixture of 50 µl of a stock solution consisting of 20 mM Tris-HCl, 0.5 M NaCl, pH 8.0, 50 % glycerol and 10 ml PBS (1X, pH 7.4, Gibco Co.) solution. For the two groups related to the composition of the present disclosure, rhHAPLN1 stock solution (20 mM acetate, pH 5.0, 8.0 M sucrose, 0.04% PS80) was diluted with phosphate-buffered saline (PBS) to adjust the rhHAPLN1 weight ratio concentration to 0.00075% (w/w), in other words, 7,500 ng/mL, and 0.0015% (w/w), in other words, 15,000 ng/mL in a total volume of 50 µL. Afterwards, the groups were each mixed in 10 ml of PBS (1X, pH 7.4, Gibco Co.) and administered to each group once a day for 1 hour by nebulization using the above nebulizer (10 ml/hour/14 times/day). The weight of each experimental animal was measured every 2 days to confirm that there was no change in weight, and a mass dosing system and aerosol chamber (Data Science International Co., Ltd.) were used to generate the fog.

The total experimental period was 23 days, and the actual administration of each concentration of recombinant human HAPLN1 protein was 10 ml once daily for 1 hour for 14 days, and the animals were dissected after anesthesia the day after the final exposure.

### 3. Result

### A. Photographic comparison of lung tissue

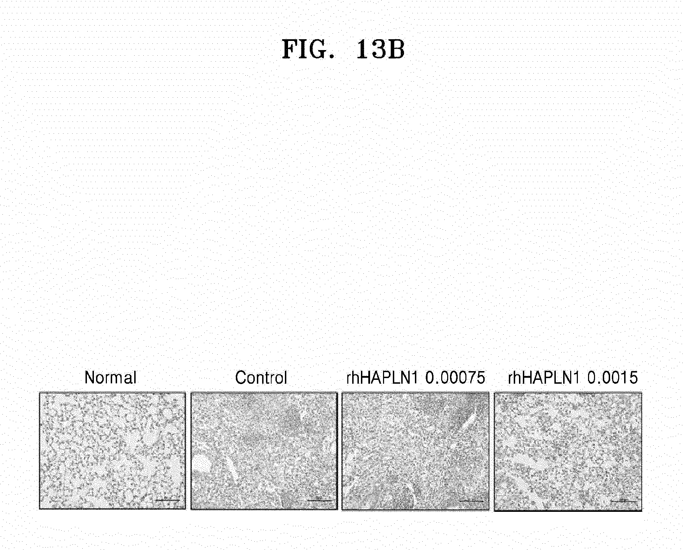

FIG. 13B is a photograph of lung tissue harvested, then the left large lobe was cut in half transversely, the upper portion was fixed in formalin, and hematoxylin and eosin (H&E) staining was performed. When looking at the photograph, it may be seen that when the composition of the present disclosure was administered compared to the control group, especially at an rhHAPLN1 weight ratio concentration of 0.0015% (w/w), the form was quite similar to that of the normal group.

### B. Therapeutic efficacy of composition of present disclosure expressed in numbers

Next, three tissue slides were prepared per mouse by cutting the middle portion of the left large lobe of the mouse, and three sections were photographed randomly on one slide, and then, for a total of nine sections, the stained darker part was distinguished from the unstained white part, and the area of the stained darker part was measured using Image J software, and each figure obtained was averaged to obtain an average value per mouse. The dark area indicate areas of advanced fibrosis.

Furthermore, the same method was repeated for the remaining three animals to obtain the average value, which was then re-averaged again to finally obtain the mean value and deviation of the area reflecting fibrosis. The p-value of significance between the normal group not treated with bleomycin and the control treated with bleomycin was 0.00038 (***p< 0.001), and the p-value of significance between the group not treated with the composition of the present disclosure (rhHAPLN1) and the group treated with 0.0015 % (w/w) rhHAPLN1 was 0.00355 (**p< 0.01), confirming statistical significance.

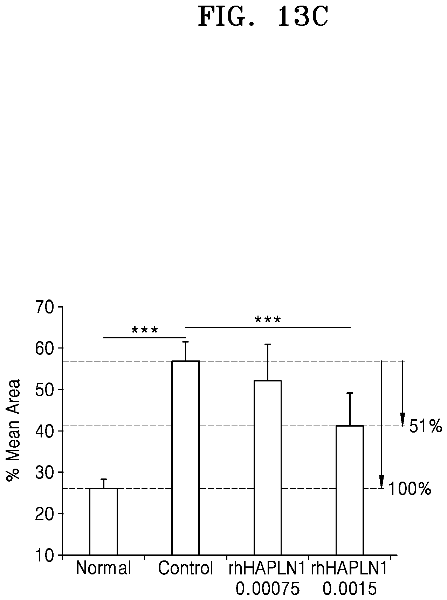

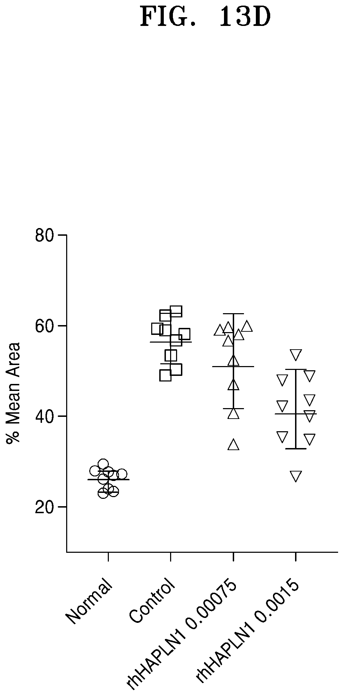

According to FIG. 13C, which shows these average values in a graph, it may be seen that when the composition of the present disclosure is administered compared to the control group, especially when the rhHAPLN1 weight ratio concentration was 0.0015%(w/w), the average value of the area of the dark area was reduced by 51 % compared the case of pulmonary fibrosis without any treatment. In other words, it was confirmed that the composition of the present disclosure exhibits an excellent therapeutic effect against pulmonary fibrosis in vivo. This is also graphed in FIG. 13D. ****p*<0.001, was calculated by Student's t-test.

### C. Therapeutic efficacy of composition of present disclosure according to Ashcroft score

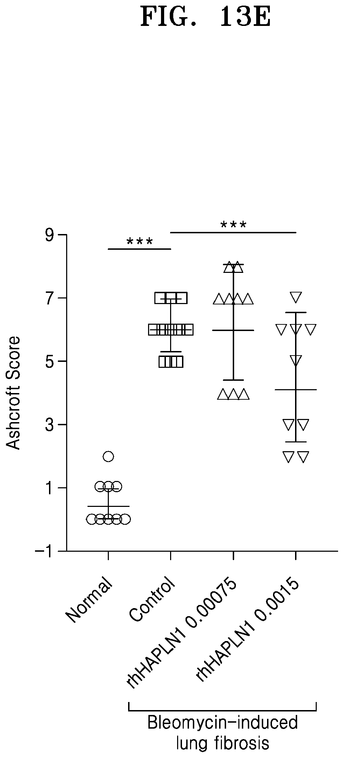

Based on the evaluation criteria as described in Example 12, three observers each scored the same data individually under the condition that they were not affected by the evaluation of others, and then each result was aggregated to determine the final average score, and the results of each evaluator were very similar, and the results of two representative observers are shown in FIG. 13E, which again confirms that the composition of the present disclosure has a preventive effect as well as an excellent therapeutic effect on pulmonary fibrosis in vivo.

### [Example 14]

### Evaluation of anti-fibrotic efficacy of composition of present disclosure using animal model inducing kidney fibrosis

An experiment was conducted to verify the therapeutic effect of the composition of the present disclosure by repeated administration to animals with acute kidney fibrosis.

### 1. Establishment of acute kidney injury induced model

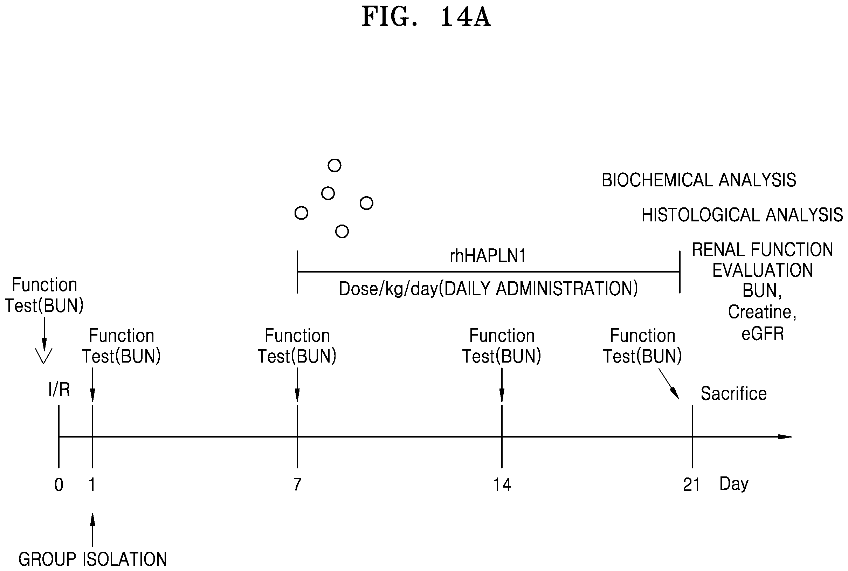

According to the experimental scheme of FIG. 14A, 8-week-old C57BL/6 male mice were subjected to pentobarbital (50 mg/kg BW, i.p.) anesthesia, a flank incision was made to induce ischemia, the kidneys were exposed, and blood flow was blocked for 25 minutes by clamping the bilateral renal pedicles using a non-traumatic microaneurysm clamp (Roboz Surgical Instruments). The clamps were then removed and the incisions were sutured. The sham-operation was performed in the same manner as the ischemia/reperfusion experiment, except for the blockage of blood flow.

One day after ischemia, blood urea nitrogen (BUN) was measured, and only mice whose value rose above about 98 mg/dL (specified as 100 mg/dL from now on) were grouped into experimental groups and the experiment was conducted. The experimental groups were administered various drugs, including the composition of the present disclosure, daily from day 7 after surgery, and the kidneys were harvested on day 14 for histologic and biochemical analysis. The drug concentrations are shown in Table 1 and the details of the experimental groups are shown in Table 2.

**[Table 1]**

| | Component | | |
|---|---|---|---|
| | rhHAPLN1 (0.2 µg/µl) | rhHAPLN1 buffer | PBS |
| rhHAPLN1 dose A (0.1 mg/kg B.W) | 12.5 µl | 0 µl | 187.5 µl |
| rhHAPLN1 dose B (0.02 mg/kg B.W) | 2.5 µl | 10 µl | 187.5 µl |
| rhHAPLN1 dose C (0.004 mg/kg B.W) | 0.5 µl | 12 µl | 187.5 µl |
| rhHAPLN1 buffer (Vehicle) | 0 µl | 12.5 µl | 187.5 µl |
| Pirfenidone (300 mg/kg B.W) | | | |

**[Table 2]**

| Experimental group (8 weeks C57BL/6 male mouse) | | Total number of animals (n) | Administratio n method |
|---|---|---|---|
| Sham-rhHAPLN1 buffer (Vehicle) | (SV) | n=5 | Intraperitone al injection |
| Sham-rhHAPLN1 dose B (0.02 mg/kg B.W) | (SB) | n=5 | Intraperitone al injection |
| IR-rhHAPLN1 buffer (Vehicle) | (IRV) | n=10 | Intraperitone al injection |
| IR-pirfenidone (300 mg/kg B.W) | (IRP) | n=10 | Oral administratio n |
| IR-rhHAPLN1 dose A (0.1 mg/kg B.W) | (IRA) | n=10 | Intraperitone al injection |
| IR-rhHAPLN1 dose B (0.02 mg/kg B.W) | (IRB) | n=10 | Intraperitone al injection |
| IR-rhHAPLN1 dose C (0.004 mg/kg B.W) | (IRC) | n=10 | Intraperitone al injection |

In addition, blood was taken on day 7 after drug administration to measure BUN concentration, and 14 days later, blood and urine were taken to measure creatinine concentration in blood and urine and BUN concentration in blood. Additionally, creatinine clearance was calculated.

### 2. Results derivation experiment

### A. Evaluation of efficacy of composition of present disclosure for reducing αSMA

### (1) Experimental method

Biochemical analysis (evaluation of fibrosis index expression): To confirm the expression of αSMA in kidney tissue, western blot was performed using antibodies against αSMA.

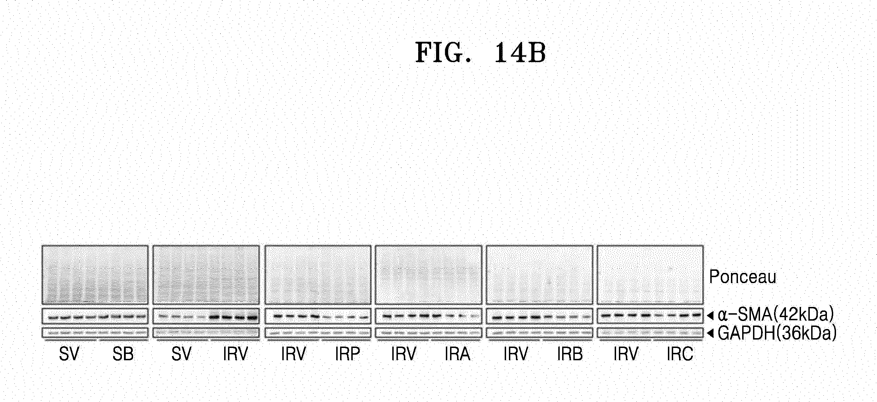

To confirm the expression of αSMA in sham-operation (Sham) and ischemia/reperfusion (IR) mouse kidney tissue, samples from each experimental group were loaded onto a single gel, western blot was performed, and the density of each band was measured. Ponceau and GADPH were utilized as equal loading markers (FIG. 14B). Where SV; sham-vehicle, SB; sham-rhHAPLN1-dissolving solution, IRV; IR-vehicle, IRP; IR-pirfenidone, IRA; IR-composition of the present disclosure (rhHAPLN1) A, IRB; IR-composition of the present disclosure (rhHAPLN1) B, IRC; IR-composition of the present disclosure (rhHAPLN1) C, respectively, and more details are shown in Table 1 and Table 2 above. More specifically, the Sham-vehicle is a group in which only the rhHAPLNB1-free buffer solution was diluted in PBS and IP-injected into the Sham control group. Sham-rhHAPLN1-dissolving solution is a group in which rhHAPLN1 concentration B (0.02mg/kg) was diluted and IP injected to confirm the effect of rhHAPLN1 under normal conditions. IR-vehicle is a buffer-treated group that does not include rhHAPLN1 in the I/R fibrosis induction model. IR-pirfenidone is a group in which 300 mg/kg of pirfenidone was administered orally after induction of I/R fibrosis.

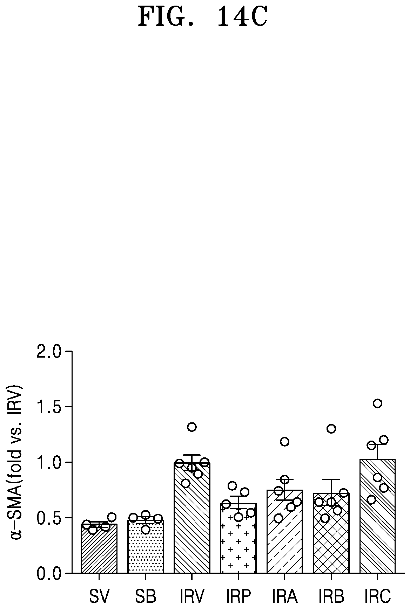

In addition, the fold change in expression level for IRV in relation to αSMA expression was graphed (FIG. 14C).

### (2) Result

The expression of αSMA, an indicator of fibrosis in the kidney, was significantly higher when ischemia/reperfusion was induced compared to sham-operation (SV and SB).

However, the expression of αSMA in the groups (IRA and IRB) administered with the composition of the present disclosure (rhHAPLN1) was lowered to pirfenidone-like levels compared to the group administered with the control drug (IRV). Here, the IRC is thought to be at an extremely low concentration and appears to be as high as the IRV.

### B. Evaluation of efficacy of composition of present disclosure for collagen reduction

### (1) Experimental method

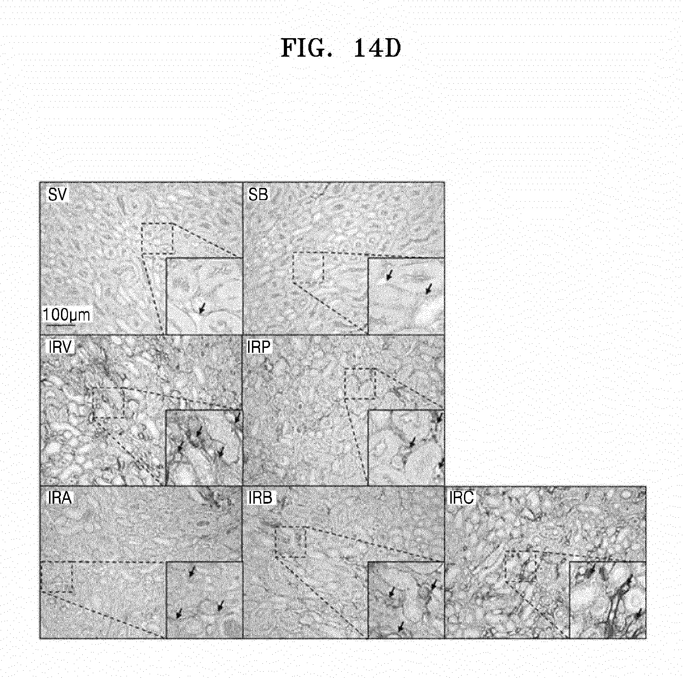

As part of the histological analysis of fibrosis, paraffin-fixed kidneys were cut into 3 µm sections, and Sirus red and PAS staining were performed on these slide sections to assess collagen expression and fibrotic lesions in the tissue, respectively (FIG. 14D). Where SV; sham-vehicle, SB; sham-rhHAPLN1-dissolving solution, IRV; IR-vehicle, IRP; IR-pirfenidone, IRA; IR-composition of the present disclosure (rhHAPLN1) concentration 0.1 mg/kg, IRB; IR- the composition of the present disclosure (rhHAPLN1) at a concentration of 0.02 mg/kg, IRC; IR-the composition of the present disclosure (rhHAPLN1) at a concentration of 0.004 mg/kg, respectively.

### (2) Result

As shown in FIG. 14D, in the group of kidneys exposed to ischemia/reperfusion (IRV), collagen-positive area (red) appeared significantly more in the renal interstitium (between tubules) than in the sham-operation group (SV and SB). In this regard, in order to more objectively confirm the anti-fibrotic efficacy of the present disclosure related to the reduction of collagen, a blinded test was performed by a total of three researchers on the tissue stained photographs in FIG. 14D. As a result, all three researchers confirmed that the expression of collagen was weaker in the experimental group treated with pirfenidone, IRP, and the groups treated with the composition of the present disclosure, IRA and IRB, compared to the IRV group.

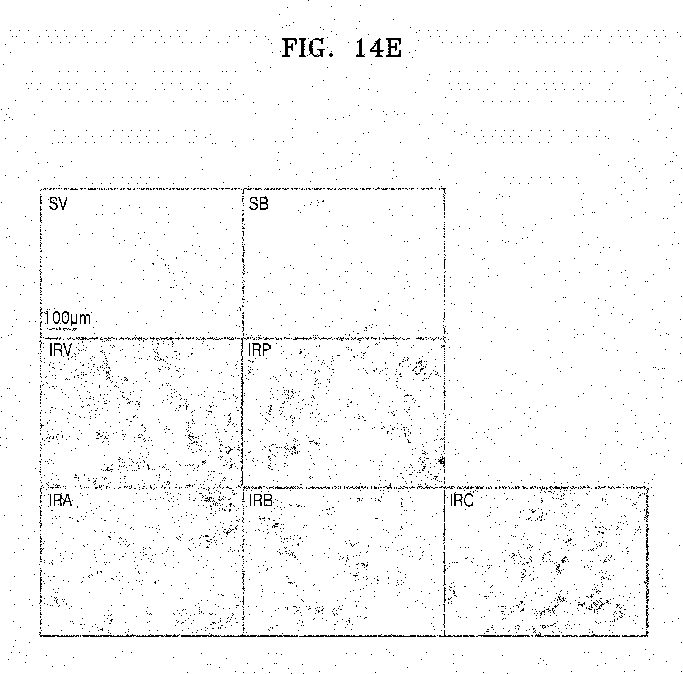

In addition, in order to quantify the expression of collagen in the above-mentioned tissue, two of the outer medulla areas of the kidney were randomly imaged in the tissue staining photograph of FIG. 14D, and collagen-positive area was marked. Here, the red color is a sirus red positive signal (FIG. 14E).

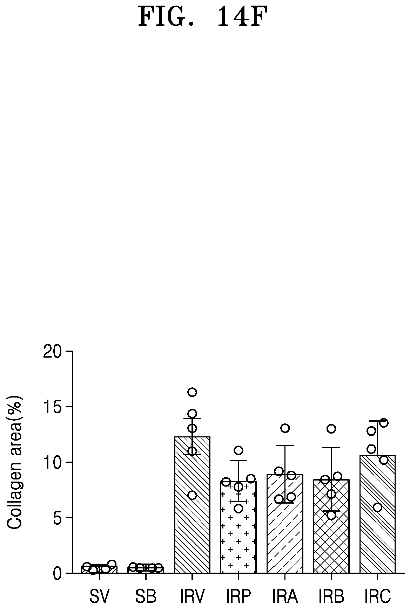

Based on this, the collagen positive area was quantified using i-Solution software (IMT), and as observed under the microscope in FIG. 14D, the collagen positive area was weaker in the IRP, IRA and IRB groups compared to the IRV group (FIG. 14F). This indicates that the composition of the present disclosure (rhHAPLN1) reduces the expression of collagen to an extent equivalent to that of pirfenidone, indicating that the composition has an anti-fibrotic efficacy in vivo.

### C. Evaluation of efficacy of composition of present disclosure on creatinine clearance

### (1) Experimental method

As an experiment to analyze kidney function, blood was taken through a glass capillary tube with heparin added through the retroorbital vein plexus, and urine was obtained using a metabolic cage. Blood urea nitrogen and creatinine (plasma creatinine, PCr) concentrations were measured using Vitros250 (Johnson & Johnson).

Creatinine clearance is a method of estimating glomerular filtration rate using the amount of creatinine removed by the kidneys, calculated as urine creatinine concentration X urine volume/Plasma creatinine concentration, which was measured on day 21 after ischemia/reperfusion in this experiment. **p*<0.05. N=4-6. * The IR experimental group used only animals in which the BUN concentration rose above 100 mg/dL on the first day after surgery, causing a similar level of damage.

### (2) Result

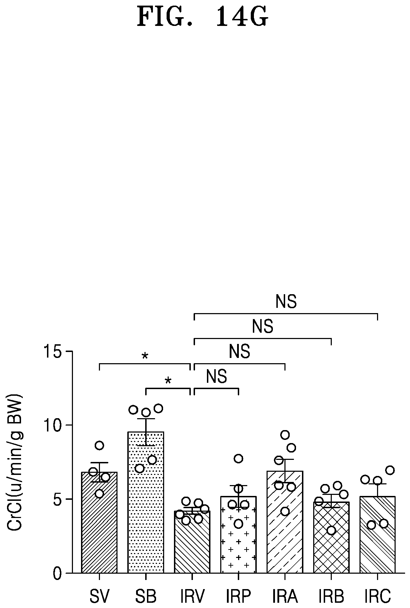

As shown in FIG. 14G, the group of kidneys exposed to ischemia/reperfusion (IRV) exhibited a significant decrease in creatinine clearance, in other words, the amount of creatinine removed from the blood by the kidneys functioning properly, compared to the corresponding groups of control animals without ischemia/reperfusion (SV and SB).

However, in the groups treated with the composition of the present disclosure (IRA, IRB, and IRC), creatinine clearance was increased, especially in the IRA group (the composition of the present disclosure administered as rhHAPLN1 at 0.1 mg/kg/BW), creatinine clearance was significantly higher compared to the rhHAPLN1 buffer (IRV) and the pirfenidone-administered group (IRP), in which the composition of the present disclosure contained no rhHAPLN1 protein but only buffer (PBS). In particular, CrCI of IRA was statistically significantly higher than that of IRV. This indicates that administration of the composition of the present disclosure substantially alleviates the decrease in renal function induced by ischemia/reperfusion injury.

### Industrial Applicability

### Sequence List Text

SEQ ID NO: 1:

MKSLLLLVLISICWADHLSDNYTLDHDRAIHIQAENGPHLLVEAEQAKVFSHRGGNVTL
PCKFYRDPTAFGSGIHKIRIKWTKLTSDYLKEVDVFVSMGYHKKTYGGYQGRVFLKGG
SDSDASLVITDLTLEDYGRYKCEVIEGLEDDTVVVALDLQGVVFPYFPRLGRYNLNFHE
AQQACLDQDAVIASFDQLYDAWRGGLDWCNAGWLSDGSVQYPITKPREPCGGQNTV
PGVRNYGFWDKDKSRYDVFCFTSNFNGRFYYLIHPTKLTYDEAVQACLNDGAQIAKV
GQIFAAWKILGYDRCDAGWLADGSVRYPISRPRRRCSPTEAAVRFVGFPDKKHKLYG
VYCFRAYN

## Claims

1. A pharmaceutical composition for preventing or treating a fibrotic disease, comprising hyaluronan and proteoglycan link protein 1 (HAPLN1) or a gene encoding the same as an active ingredient.

2. The composition of claim 1, wherein the protein has at least 80 % sequence identity to an amino acid sequence of SEQ ID NO: 1.

3. The composition of claim 1, wherein a nucleic acid for the gene is contained in an expression vector.

4. The composition of claim 1, wherein a foci of fibrotic disease is selected from the group consisting of skin, liver, intestine, heart, lung, and kidney.

5. The composition of claim 1, wherein a foci of fibrotic disease is selected from the group consisting of a skin fibroblast, hepatic stellate cell, colon fibroblast, cardiac microvascular endothelial cell, lung fibroblast, kidney renal tubule cell, and renal proximal tubule epithelial cell.

6. The composition of claim 1, wherein the fibrotic disease is ischemic fibrosis.

7. The composition of claim 1, wherein a single dose of the composition is from 0.1 ng/ml to 500 ng/ml.

8. The composition of claim 1, wherein the composition is administered at a dose of 0.001 mg/kg BK to 5 mg/kg BK of rhHAPLN1 protein when administered in vivo.

9. The composition of claim 1, wherein the composition is comprised as a major or minor active ingredient in a composition for preventing or inhibiting fibrosis of cells.

10. A method of preventing or inhibiting fibrosis of a cell by treating the cell with a composition comprising hyaluronan and proteoglycan link protein 1 (HAPLN1) or a gene encoding the same as an active ingredient.

11. A kit for preventing or inhibiting fibrosis of cells, comprising a composition according to any one of claims 1 to 9 and instructions for treatment according to the method of claim 10.

12. An experimental reagent composition related to preventing or inhibiting fibrosis of cells, comprising a composition according to any one of claims 1 to 9.
